# EUROPÄISCHE PATENTANMELDUNG

(11) **EP 2 316 338 A1**
(43) Veröffentlichungstag der Anmeldung: **04.05.2011**
(21) Anmeldenummer: 09174764.2
(22) Anmeldetag: 02.11.2009
(51) Int. Cl.: A61B 5/15

(54) **Lanzettengreifer zum Einsatz in einer Lanzettenvorrichtung**

(71) Anmelder: Roche Diagnostics GmbH, 68305 Mannheim (DE); F. Hoffmann-La Roche AG, 4070 Basel (CH)
(72) Erfinder: Die Erfindernennung liegt noch nicht vor
(74) Vertreter: Stößel, Matthias

(57) **Zusammenfassung**

Es wird eine Greifvorrichtung (112) zum Einsatz in einer Lanzettenvorrichtung (110) zur Gcncricrung einer Probe einer Körperflüssigkeit vorgeschlagen. In der Lanzcttcnvorrichtung (110) ist eine Mehrzahl von Lanzetten (120) nacheinander auf einem Trägerelement (116) in einer Applikationsposition (124) bcrcitstcllbar. Die Greifvorrichtung (112) ist eingerichtet, um in der Applikationsposition (124) jeweils eine Lanzette (120) zu erfassen und eine Stechbewegung mit der Lanzette (120) durchzuführen. Die Greifvorrichtung (112) weist mindestens ein Stoppelement (144) auf, welches eingerichtet ist, um die Lanzette (120) in der Applikationsposition (124) zu stoppen und eine Weiterbewegung des Trägerelements (116) vorübergehend zu verhindern. Die Greifvorrichtung (112) weist weiterhin mindestens ein Positionierungselement (148) auf, welches eingerichtet ist, um die Lanzette (120) während der Stechbewegung in mindestens einer Richtung quer zu einer Stechrichtung (126) zu positionieren.

## Beschreibung

### Gebiet der Erfindung

Die Erfindung betrifft eine Greifvorrichtung, welche als Lanzettengreifer in einer Lanzettenvorrichtung zur Generierung einer Probe einer Körperflüssigkeit eingesetzt werden kann. Weiterhin betrifft die Erfindung eine Lanzettenvorrichtung zur Generierung einer Probe einer Körperflüssigkeit. Derartige Greifvorrichtungen und Lanzettenvorrichtungen können im Bereich der medizinischen Diagnostik eingesetzt werden, um einfach und schnell Körperflüssigkeitsproben zu generieren, zum Zwecke einer nachfolgenden Analyse und/oder Diagnose. Beispielsweise können mittels der Lanzenvorrichtung Proben von Blut und/oder interstitieller Flüssigkeit erzeugt werden, zum Zwecke eines qualitativen und/oder quantitativen Nachweises mindestens eines Analyten in der Probe, beispielsweise mindestens eines Metaboliten. Ohne Beschränkung weiterer möglicher Anwendung wird die Erfindung im Nachfolgenden im Wesentlichen in Bezugnahme auf eine Blutglukose-Analyse beschrieben. Auch andere Anwendungen sind jedoch möglich.

### Stand der Technik

Die Untersuchung von Blutproben oder anderen Proben von Körperflüssigkeiten, wie beispielsweise interstitieller Flüssigkeit, ermöglicht das frühzeitige und zuverlässige Erkennen von pathologischen Zuständen sowie die gezielte und fundierte Kontrolle von Körperzuständen. Die medizinische Diagnostik setzt in der Regel die Gewinnung einer Probe aus Blut oder interstitieller Flüssigkeit des zu untersuchenden Individuums voraus. Zur Gewinnung der Probe kann die Haut, beispielsweise an der Fingerbeere oder dem Ohrläppchen der zu untersuchenden Person, mit Hilfe einer sterilen, spitzen oder scharfen Lanzette perforiert werden, um so einige wenige Mikroliter Blut oder weniger für die Analyse zu gewinnen. Insbesondere eignet sich diese Methode für die Analyse einer Probe, die unmittelbar nach der Probengewinnung durchgeführt wird.

Vor allem im Bereich des sogenannten "Home-Monitorings", also dort, wo medizinische Laien selbst einfache Analysen des Bluts oder der interstitiellen Flüssigkeit durchführen, und dort für insbesondere für die regelmäßige, mehrmals täglich durchzuführende Blutgewinnung durch Diabetiker für die Kontrolle der Blutglukose-Konzentration, werden Lanzetten und dazu passende Geräte angeboten. Die Geräte, welche auch als Stechhilfen bezeichnet werden, sollen eine möglichst schmerzarme und reproduzierbare Probengewinnung ermöglichen.

Neben Stechhilfen, in welchen einzelne Lanzetten eingesetzt werden, die miteinander nicht durch ein gemeinsames Trägerelement verbunden sind, sind aus dem Stand der Technik auch Bandgeräte für Mehrfach-Stechhilfen bekannt, in welchen eine Mehrzahl von Lanzetten magaziniert und über ein gemeinsames Trägerband verbunden ist. So beschreibt die DE 28 03 354 B1 ein Blutprobenentnahmegerät mit einer unter Krafteinwirkung gegen die Körperoberfläche eines Patienten bewegbaren Nadel sowie einer geeigneten Betätigungseinrichtung mit einem Stößel und einem Auslöser. Dabei werden Blutlanzetten als Nadel verwendet, welche einzeln in Taschen einer Streifenverpackung aufgenommen sind und mittels einer an der Streifenverpackung angreifenden Transporteinrichtung in das Blutprobenentnahmegerät eingeführt werden. Dabei wird eine Transporteinrichtung in Form einer Trommel mit Mitnehmerstiften offenbart, welche zum Bereitstellen der Blutlanzetten schrittweise weitergetaktet wird.

Aus EP 1360935 A1 ist ein kontinuierlicher Streifen von Testelementen bekannt. Diese sind jeweils individuell in einer wasserundurchlässigen Verpackung aufgenommen und umfassen eine Mikronadel mit einem integrierten Teststreifensensor. Dabei wird ein Rollenantrieb vorgeschlagen, wobei der Streifen eine Vielzahl von äquidistanten Löchern aufweist, in welche Transportstifte eingreifen. Auf diese Weise wird der Streifen jeweils um einen festen Betrag weitergetaktet.

WO2005/104948A1 beschreibt ein Testmagazin mit zwei sandwichartig miteinander verbundenen, aufwickelbaren Folienbändern, zwischen denen Aufnahmezellen für Testelemente freigehalten sind. Stecheinheiten und Testeinheiten sind dabei jeweils in gesonderten Aufnahmezellen getrennt voneinander angeordnet. Ist es eine Bandzugvorrichtung mit zwei Wickelspulen vorgesehen, welche die beiden Folienbänder auseinanderreißt, um die Stecheinheiten nacheinander bereitzustellen.

Aus WO2005/107596A2 ist ein Vorrat von Lanzetten für ein Vielfach-Lanzettengerät bekannt. Die Lanzetten werden auf einem Band transportiert und nacheinander von einer Speicherposition in eine Applikationsposition gebracht, indem das Band um eine Umlenkung transportiert wird. Mittels eines entsprechenden Aktormechanismus wird in der Applikationsposition das Band bewegt, sodass die in der Applikationsposition befindliche Lanzette mit samt dem Band eine Stechbewegung durchführt.

Aus WO2008/125178A1 ist ein Stechsystem zum Gewinnen einer Körperflüssigkeitsprobe bekannt, welches mindestens ein Magazin mit einem Lanzettenträger und mehreren Lanzetten enthält. Weiterhin wird ein Stechgerät mit einem Fach für ein derartiges Magazin offenbart, welches einen Fortschaltmechanismus aufweist, um die in dem Magazin enthaltenen Lanzetten nacheinander in eine Stechposition zu bringen. Weiterhin ist ein Stechantrieb vorgesehen, der eine in der Stechposition positionierte Lanzette für eine Stechbewegung beschleunigt. Mittels eines Fortschaltmechanismus wird eine Rolle jeweils so weit gedreht, dass für einen Stich eine frische Lanzette des Lanzettenträgers in die Stechposition gelangt.

Diese bekannten Lanzettenvorrichtungen, bei denen Lanzetten auf einem Band magaziniert sind, weisen jedoch in der Praxis verschiedene Schwierigkeiten und Herausforderungen auf. Insbesondere stellt die genaue Positionierung der Lanzetten in der Applikationsposition eine technische Herausforderung dar, insbesondere da bei der Herstellung der Lanzettenbänder in gewissem Rahmen Toleranzen auftreten können. Die aus dem Stand der Technik bekannten Positionierungen basieren jedoch in der Regel auf einer äquidistanten Weitertaktung des Lanzettenbands, in vielen Fällen unterstützt durch Positionierungslöcher auf dem Lanzettenband. Diese Mechanismen berücksichtigen jedoch mögliche Toleranzen der Positionierung der Lanzetten auf den Lanzettenbändern nicht in ausreichendem Maße.

Aus WO2009/037341 ist daher ein Kombinationsantrieb für ein Probengewinnungssystem zum Gewinnen einer flüssigen Probe bekannt. Unter anderem wird dabei vorgeschlagen, einen Bandtransport eines Testelementbands erst zu stoppen, wenn von einem entsprechenden Mechanismus eine Dickenänderung der Dicke des Analysebands in einer Applikationsposition erkannt wird. Zu diesem Zweck wird beispielsweise ein Greifer vorgelschlagen, welcher die Dicke des Analysebands abtastet. Stößt beispielsweise eine bestimmte, zu verwendende Lanzette gegen eine dafür vorgesehene Kante des Greifers, so wird eine Gegenkraft auf das Analyseband ausgeübt, welche einen Weitertransport des Analysebands stoppt. Der Greifer wirkt somit als Blockadeelement. Nach einer Verwendung der Lanzette kann der Greifer automatisch oder manuell kurzfristig geöffnet oder auf andere Weise freigegeben werden, um einen Weitertransport des Analysebands zu ermöglichen. Der Greifer kann beispielsweise als federbelasteter Greifer ausgestaltet sein, um genügend Kraft auf das Analyseband auszuüben, sodass das Analyseband gestoppt werden kann.

Der in WO2009/037341 A1 beschriebene Greifer stellt somit eine lösbare Stoppfunktion bereit, welche eine individuelle Positionierung der Lanzetten auf dem Analyseband erkennt. Allerdings sind derartige lösbare Stoppfunktionen in der Praxis vergleichsweise Bauraumaufwendig, was sich insbesondere bei kompakten, transportablen Lanzettenvorrichtungen oder Lanzettenvorrichtungen, welche in ein Analysegerät mit zusätzlicher Analysefunktion integriert sind, nachteilig bemerkbar macht. Eine weitere technische Herausforderung einfacher Greifer mit Stoppfunktion besteht darin, dass die Stechbewegung in vielen Fällen vergleichsweise unkontrolliert erfolgt, da sich, beispielsweise aufgrund einer Bandspannung des Trägerbands, die Lanzetten in der Applikationsposition verbiegen können, wodurch eine definierte und gleichmäßige Stechbewegung beeinträchtigt werden kann.

### Aufgabe der Erfindung

Es ist daher eine Aufgabe der vorliegenden Erfindung, eine Greifvorrichtung sowie eine Lanzettenvorrichtung bereitzustellen, welche die Nachteile bekannter Greifvorrichtungen und Lanzettenvorrichtungen zumindest weitgehend vermeiden. Insbesondere soll ein Toleranzausgleich der Positionierung der Lanzetten auf dem Trägerelement ermöglicht werden, bei gleichzeitig hoher Integrationsdichte und geringem Bauraum sowie einer gleichmäßigen und reproduzierbaren Stechbewegung.

### Offenbarung der Erfindung

Diese Aufgabe wird durch eine Greifvorrichtung sowie eine Lanzettenvorrichtung mit den Merkmalen der unabhängigen Patentansprüche gelöst. Vorteilhafte Weiterbildungen der Erfindung, welche einzeln oder in Kombination realisierbar sind, sind in den abhängigen Patentansprüchen dargestellt.

In einem ersten Aspekt der Erfindung wird eine Greifvorrichtung zum Einsatz in einer Lanzettenvorrichtung zu Generierung einer Probe von Körperflüssigkeit vorgeschlagen. In einem zweiten Aspekt wird eine entsprechende Lanzettenvorrichtung zur Generierung einer Probe einer Körperflüssigkeit vorgeschlagen, welche eine erfindungsgemäße Greifvorrichtung in einer oder mehreren der im Folgenden beschriebenen Ausgestaltungen umfasst. Für mögliche Ausgestaltungen der Lanzettenvorrichtung kann daher auf die Beschreibung der Greifvorrichtung verwiesen werden und umgekehrt. Die Lanzettenvorrichtung kann insbesondere als Stechhilfe ausgestaltet sein oder Bestandteil einer Stechhilfe sein. Alternativ kann die Lanzettenvorrichtung jedoch auch Bestandteil eines integrierten Testgeräts sein, welches neben einer Lanzettenfunktion weitere Funktionen umfassen kann, beispielsweise eine oder mehrere Analysefunktionen.

Die Lanzettenvorrichtung ist derart eingerichtet, dass in dieser eine Mehrzahl von Lanzetten nacheinander auf einem Trägerelement in einer Applikationsposition bereitstellbar sind. Unter einer Applikationsposition ist dabei eine Position zu verstehen, in welcher mittels mindestens einer in dieser Position bereitgestellten Lanzette eine Stechbewegung durchführbar ist, um eine Hautpartie eines Benutzers zu perforieren. Beispielsweise kann die Lanzettenvorrichtung zu diesem Zweck einen entsprechenden Aktuationsmechanismus aufweisen, welcher unten exemplarisch noch näher beschrieben wird.

Unter einer Lanzette ist dabei allgemein ein Element zu verstehen, mittels dessen eine Perforation einer Hautpartie zur Gewinnung einer Probe der Körperflüssigkeit möglich ist. Beispielsweise können die Lanzetten zu diesem Zweck eine Spitze und/oder eine Schneide aufweisen. Verschiedene Arten von Lanzetten sind dabei realisierbar. Beispielsweise können Rundlanzetten mit Nadelelementen verwendet werden. Alternativ oder zusätzlich können auch Flachlanzetten verwendet werden, also Lanzetten, welche beispielsweise aus einem Metallband ausgestanzt, ausgeschnitten, heraus geätzt, oder auf ähnliche Weise erzeugt sind. Die Lanzetten können eine reine Stechfunktion oder Schneidefunktion ausüben, um die Hautpartie zu perforieren. Alternativ können die Lanzetten auch zusätzliche Funktionen übernehmen, wie beispielsweise Probenahmenfunktionen. So können die Lanzetten beispielsweise jeweils ein oder mehrere Kapillarelemente umfassen, um eine Probe der Körperflüssigkeit aus dem Körpergewebe des Benutzers heraus zu transportieren, beispielsweise zu einem Testfeld. Dementsprechend können die Lanzetten beispielsweise als Microsampler ausgestaltet sein, also als Lanzettenelemente mit integriertem Kapillarelement, beispielsweise in Form mindestens eines Kapillarspalts. Weiterhin können die Lanzetten ein oder mehrere Testelemente umfassen, beispielsweise ein oder mehrere Testfelder, beispielsweise mit einer Testchemie, welche eine oder mehrere physikalische und/oder chemische Eigenschaften bei Anwesenheit des mindestens einen nachzuweisenden Analyten ändert. Verschiedene Ausgestaltungen sind möglich.

Unter einem Trägerelement ist grundsätzlich ein beliebiges, vorzugsweise kontinuierliches Element zu verstehen, welches die Mehrzahl der Lanzetten miteinander verbindet. Beispielsweise kann das Trägerelement als Trägerband ausgestaltet sein. So kann das Trägerband beispielsweise eine oder mehrere bandförmige Träger umfassen, beispielsweise aus einem Kunststoffmaterial und/oder einem Papiermaterial. Beispielsweise können die Lanzetten im Wesentlichen - das heißt abgesehen von Positionstoleranzen - äquidistant auf dem Trägerelement positioniert sein. Beispielsweise kann das Trägerelement eine Längserstreckungsrichtung, welche beispielsweise einer Transportrichtung des Trägerelements entsprechen kann. Die Lanzetten sind vorzugsweise derart relativ zu dem Trägerelement positioniert, dass die Lanzettenspitzen quer zur Längserstreckungsrichtung des Trägerelements, beispielsweise quer zur Bandrichtung, verlaufen, beispielsweise im Wesentlichen senkrecht zur Bandrichtung. Das Trägerelement kann beispielsweise flexibel ausgestaltet sein. Dementsprechend kann das Trägerelement beispielsweise durch eine entsprechende Führung geführt werden, beispielsweise mit einer geeigneten Umlenkung. Beispielsweise kann in der Applikationsposition das Trägerelement derart umgebogen werden, dass die Lanzettenspitze der in der Applikationsposition befindlichen Lanzette freigelegt wird, um eine Stechbewegung durchzuführen.

Das Trägerelement kann beispielsweise als Trägerband ausgestaltet sein und kann beispielsweise mittels eines oder mehrerer Wickel in der Lanzettenvorrichtung aufgenommen sein. Beispielsweise kann die Lanzettenvorrichtung einen Gutwickel zur Bereitstellung unverbrauchter Lanzetten sowie einem Schlechtwickel zum Aufwickeln von Trägerelement-Abschnitten mit bereits benutzen Lanzetten aufweisen. Der Gutwickel und der Schlechtwickel können beispielsweise fest in der Lanzettenvorrichtung integriert sein. Alternativ können diese jedoch auch auswechselbar ausgestaltet sein, beispielsweise indem diese Bestandteil einer in die Lanzettenvorrichtung einlegbaren, auswechselbaren Bandkassette sind. Die Lanzettenvorrichtung kann insbesondere einen Transportmechanismus zum Weitertransportieren des Trägerelements aufweisen, beispielsweise einen entsprechenden Antrieb zum Antreiben des Schlechtwickels zum Abspulen des Trägerelements von dem Gutwickel. Es wird darauf hingewiesen, dass grundsätzlich alternativ zu einer Ausgestaltung des Trägerelements als Trägerband auch andere Ausgestaltungen in Betracht kommen, beispielsweise Ausgestaltungen mit einer Gliederkette oder einer anderen Art von Trägerelement, durch welches die Mehrzahl der Lanzetten miteinander verbunden wird. Besonders bevorzugt ist jedoch die Ausgestaltung als Trägerband, sodass das Trägerband gemeinsam mit den Lanzetten ein Lanzettenband bildet.

Zusätzlich zu den Lanzetten kann das Trägerelement auch ein oder mehrere weitere Elemente umfassen oder aufnehmen. Beispielsweise kann das Trägerelement ein oder mehrere Testelemente umfassen und/oder es können ein oder mehrere Testelemente mit dem Trägerband verbunden sein. Beispielsweise kann jeweils einer Lanzette jeweils ein Testelement zugeordnet sein, beispielsweise integriert in die Lanzette oder beabstandet zu der Lanzette aufgenommen auf dem Trägerelement, beispielsweise dem Trägerband. Die Testelemente können beispielsweise, wie oben dargestellt, jeweils ein oder mehrere Testfelder umfassen, beispielsweise mit einer Testchemie zum qualitativen und/oder quantitativen Nachweis des mindestens einen Analyten. Auch eine Ausgestaltung als reines Lanzettenband mit bevorzugt äquidistant angeordneten Lanzetten ist jedoch grundsätzlich möglich. Alternativ oder zusätzlich ist jedoch auch eine nicht-äquidistante Anordnung möglich. Vorzugsweise verbleiben die Lanzetten nach dem Stechvorgang auf dem Trägerelement, sodass diese mit dem Trägerelement entsorgt werden können. Das Trägerelement kann beispielsweise, wie oben beschrieben, auf einem Schlechtwickel aufgewickelt werden. Das Trägerelement und die Lanzetten können fest miteinander verbunden werden, sodass das Trägerelement beim Stechvorgang zumindest in der Applikationsposition mit bewegt wird.

Derartige Lanzettenvorrichtungen, mit Ausnahme der im Folgenden näher beschriebenen Greifvorrichtung, können grundsätzlich den aus dem Stand der Technik bekannten Lanzettenvorrichtungen entsprechen. Insbesondere kann die Lanzettenvorrichtung, mit Ausnahme der im Folgenden beschriebenen Greifvorrichtung, ganz oder teilweise ausgestaltet sein wie das in WO2009/037341A1 beschriebene Probengewinnungssystem und/oder kann ein derartiges Probengewinnungssystem umfassen. Dementsprechend kann das Probengewinnungssystem eine Kopplungselement und eine Antriebseinheit umfassen. Für weitere mögliche Details kann auf die WO2009/037341A1 verwiesen werden. Auch andere Ausgestaltungen der Lanzettenvorrichtung sind jedoch grundsätzlich möglich.

Die Greifvorrichtung ist zum Einsatz in der beschriebenen Lanzettenvorrichtung ausgestaltet. Die Greifvorrichtung ist eingerichtet, um in der Applikationsposition jeweils eine Lanzette zu erfassen und eine Stechbewegung mit der Lanzette durchzuführen. Unter einer Stechbewegung ist dabei eine Bewegung zu verstehen, welche hin zu einer Hautoberfläche eines Benutzers gerichtet ist, vorzugsweise gefolgt von einer Rückbewegung. Beispielsweise kann diese Stechbewegung senkrecht zu einer Längserstreckungsrichtung des Trägerelements, beispielsweise des Trägerbands, gerichtet sein. Die Stechbewegung kann dabei beispielsweise eine Einstichtiefe zwischen einigen zehn Mikrometern bis hin zu einigen Millimetern umfassen, vorzugsweise zwischen hundert Mikrometern und einem Millimeter. Die Stechbewegung kann insbesondere sehr schnell durchgeführt werden, beispielsweise mit einer Geschwindigkeit von einigen Metern pro Sekunde. Alternativ oder zusätzlich kann die Greifvorrichtung jedoch auch vorgesehen sein, um mindestens ein Testelement zu ergreifen und mit diesem mindestens eine Probennahmebewegung durchzuführen, beispielsweise um mit einem Testfeld des Testelements eine Probe von einer Hautoberfläche eines Benutzers abzuholen. Wiederum alternativ kann zu diesem Zweck auch eine separate Greifvorrichtung zusätzlich vorgesehen sein.

Die Greifvorrichtung weist mindestens ein Stoppelement auf, welches eingerichtet ist, um die Lanzette in der Applikationsposition zu stoppen und eine Weiterbewegung des Trägerelements vorübergehend zu verhindern. Dies bedeutet, dass das Stoppelement derart mit dem Trägerelement und den darauf aufgebrachten Lanzetten zusammenwirkt, dass bei einem Transport des Trägerelements jeweils, vorzugsweise automatisch, eine in der Applikationsposition ankommende Lanzette gestoppt wird und in der Applikationsposition gehalten wird. Beispielsweise kann dieses Stoppelement, wie unten noch näher ausgeführt wird, einen Anschlag umfassen, gegen welchen die Lanzette direkt oder indirekt, d.h. unter Zwischenschaltung eines oder mehrerer Elemente, stößt, sobald diese die Applikationsposition erreicht. Auf diese Weise kann beispielsweise eine Kraft auf das Trägerelement ausgeübt werden, welche ausreichend ist, um eine Weiterbewegung des Trägerelements zu verhindern. Beispielsweise kann diese Kraft eine maximale Kraft einer Rutschkupplung eines Antriebs des Trägerelements, beispielsweise eines Antriebs des Schlechtwickels, übersteigen, sodass ein Weitertransport des Trägerelements, beispielsweise des Trägerbands, verhindert wird. Das Stoppelement kann somit beispielsweise auf Dickenänderungen eines Lanzettenbands reagieren und aufgrund dieser Dickenänderung, ausgelöst durch eine Applikationsposition ankommende Lanzette, das Trägerelement an einer Weiterbewegung hindern.

Weiterhin weist die Greifvorrichtung mindestens ein Positionierungselement auf. Dieses Positionierungselement, welches vorzugsweise zumindest teilweise getrennt von dem Stoppelement ausgebildet sein kann, welches jedoch auch ganz oder teilweise integriert mit dem Stoppelement ausgebildet sein kann, ist eingerichtet, um die Lanzette während der Stechbewegung in mindestens einer Richtung quer zu einer Stechrichtung zu positionieren. Unter einer Stechrichtung ist dabei eine Richtung der Stechbewegung zu verstehen, welche, wie oben dargestellt, vorzugsweise senkrecht zu einer Transportrichtung und/oder einer Längserstreckungsrichtung des Trägerelements erfolgt. Unter einer Richtung quer zur Stechrichtung ist dabei eine Richtung unter einem Winkel zu dieser Stechrichtung zu verstehen, vorzugsweise unter einem Winkel im Wesentlichen senkrecht zur Stechrichtung, wobei jedoch auch Abweichungen von der Senkrechten möglich sind, vorzugsweise um nicht mehr als 20°, insbesondere um nicht mehr als 10°.

Beispielsweise können durch die Stechrichtung und eine Längserstreckungsrichtung des Trägerelements im Bereich der Applikationsposition eine Stechebene definiert werden. Beispielsweise kann die Lanzette durch das Positionierungselement während der Stechbewegung in der Stechebene oder in einer Ebene parallel zur Stechebene positioniert und dort insbesondere stabilisiert werden. Auf diese Weise kann beispielsweise verhindert werden, dass sich die Lanzette während der Stechbewegung schräg zur Stechebene stellt, beispielsweise in Folge einer Bandspannung des Lanzettenbands.

Beispielsweise kann durch die Längserstreckungsrichtung des Trägerelements im Bereich der Applikationsposition eine x-Richtung definiert werden. Durch die Stechrichtung selbst kann eine y-Richtung definiert werden. Die Stechebene stellt in diesem Koordinatensystem die x-y-Ebene dar. Die Positionierungsvorrichtung kann beispielsweise eingerichtet sein, um die Lanzette während des Stechvorgangs in dieser Stechebene oder in einer zu dieser Stechebene parallelen Ebene zu halten. Eine Richtung senkrecht zur Stechebene kann beispielsweise als z-Richtung definiert werden. Die Positionierungsvorrichtung kann beispielsweise somit derart eingerichtet sein, dass die z-Koordinate der Lanzette und/oder mindestens eines Punktes der Lanzette während der Stechbewegung konstant gehalten wird.

Das Positionierungselement kann insbesondere mindestens einen Niederhalter aufweisen. Unter einem Niederhalter ist dabei ein Element zu verstehen, welches eingerichtet ist, um eine Kraft auf die Lanzette senkrecht zu einer durch die Längserstreckungsrichtung des Trägerelements im Bereich der Applikationsposition und durch die Stechrichtung gebildeten Ebene, also der Stechebene, auszuüben. Beispielsweise kann der Niederhalter zusammenwirken mit einem Gegenelement, gegen welches das Trägerelement und/oder die Lanzette in der Applikationsposition gepresst werden. Die Greifvorrichtung kann insbesondere ein Greiferunterteil aufweisen, wobei die Greifvorrichtung eingerichtet ist, um das Trägerelement, beispielsweise das Trägerband mit den darauf aufgebrachten Lanzetten, zwischen dem Greiferunterteil und dem Positionierungselement zu führen. Das Greiferunterteil kann beispielsweise mindestens eine Auflagefläche umfassen, auf welches das Trägerelement geführt wird. Beispielsweise kann es sich dabei um eine ebene Auflagefläche oder eine gekrümmte Auflagefläche handeln. Das Positionierungselement, beispielsweise der Niederhalter, kann dann eingerichtet sein, um das Trägerelement und vorzugsweise die Lanzette in der Applikationsposition mit einer Kraft in Richtung des Greiferunterteils zu beaufschlagen. Beispielsweise kann der Niederhalter eingerichtet sein, um das Trägerelement, beispielsweise das Trägerband, und/oder die Lanzette in der Applikationsposition gegen das Greiferunterteil zu pressen, um die z-Koordinate gemäß der obigen Definition während des Stechvorgangs konstant zu halten.

Das Stoppelement der Greifvorrichtung kann insbesondere lösbar ausgestaltet sein. Dies bedeutet, dass das Stoppelement in seiner Position und/oder seiner Orientierung und/oder seiner Form verändert werden kann, um nach Durchführung des Stechvorgangs derart gelöst zu werden, dass eine Weiterbewegung des Trägerelements wieder ermöglicht wird. Alternativ zu dieser bevorzugten Ausgestaltung des Stoppelements als lösbares Stoppelement käme grundsätzlich auch die Möglichkeit in Betracht, die in der Applikationsposition befindliche Lanzette nach Durchführung des Stechvorgangs derart zu bewegen, dass diese freigesetzt wird.

Das Stoppelement kann insbesondere mindestens einen Anschlag aufweisen. Dieser Anschlag kann beispielsweise mindestens eine Anschlagskante umfassen, vorzugsweise eine Mehrzahl von Anschlagskanten. Diese Anschlagskanten können beispielsweise senkrecht zu der Stechebene positioniert sein. Sind mehrere Anschlagskanten vorhanden, so sind diese vorzugsweise in einem Winkel zueinander positioniert. Der Anschlag kann insbesondere benachbart zu dem Trägerelement positionierbar sein und derart ausgestaltet sein, dass bei einer Bewegung des Trägerelements in einer Transportrichtung des Trägerelements eine Lanzette an diesem Anschlag gestoppt wird. Dabei kann die Lanzette unmittelbar an diesem Anschlag anstoßen. Alternativ kann die Lanzette jedoch auch mittelbar durch diesen Anschlag gestoppt werden. So kann die Lanzette beispielsweise durch mindestens eine Folie abgedeckt sein, so dass nicht direkt die Lanzette an dem Anschlag anstößt, sondern die Folie. In diesem Fall kann beispielsweise die Dickenänderung des Lanzettenbandes, welche aufgrund der ankommenden Lanzette auftritt, vom Anschlag erfasst werden und die Stoppfunktion ausüben.

Neben der Stoppfunktion kann der Anschlag auch weitere Funktionen aufweisen, beispielsweise die Funktion, die Lanzette zusätzlich zu dem Positionierungselement auszurichten, sodass beispielsweise herstellungsbedingte Verkippungstoleranzen der Lanzette auf dem Trägerelement ausgeglichen werden können. So kann beispielsweise bei der Herstellung eine Verkippung der Lanzetten um eine Achse senkrecht zur Stechebene auftreten. Wird mindestens eine Anschlagskante verwendet, so kann diese Verkippung zumindest teilweise ausgeglichen werden und die Lanzette wieder parallel zur Stechrichtung orientiert werden.

Insbesondere wenn das Stoppelement als lösbares Stoppelement ausgestaltet ist, ist es bevorzugt, den Anschlag in der Richtung quer zu einer Längserstreckungsrichtung des Trägerelements in der Applikationsposition beweglich auszugestalten, um das Stoppelement zu lösen. Beispielsweise kann der Anschlag im Wesentlichen senkrecht zur Längserstreckungsrichtung des Trägerelements beweglich ausgestaltet sein, vorzugsweise mit einer Abweichung von nicht mehr als zwanzig Grad und besonders bevorzugt von nicht mehr als zehn Grad senkrecht zu der Längserstreckungsrichtung. Vorzugsweise ist der Anschlag auch im Wesentlichen senkrecht zur Stechrichtung beweglich, sodass der Anschlag insgesamt vorzugsweise im Wesentlichen senkrecht zur Stechebene beweglich ausgestaltet ist, um das Stoppelement zu lösen. Auch hier sind wiederum Abweichungen von der Senkrechten möglich, vorzugsweise wiederum um nicht mehr als zwanzig Grad und besonders bevorzugt um nicht mehr als zehn Grad. So kann beispielsweise zur Erfüllung der Stoppfunktion der Anschlag zunächst zumindest teilweise in Stechebene positioniert werden, um eine Lanzette in der Applikationsposition zu stoppen. Nach dem Stechvorgang kann dann der Anschlag aus dieser Ebene heraus bewegt werden, um das Stoppelement zu lösen und die Lanzette für den Weitertransport des Trägerelements freizugeben, sodass eine neue Lanzette in die Applikationsposition befördert werden kann.

Wie oben ausgeführt, umfasst die erfindungsgemäße Greifvorrichtung mindestens ein Stoppelement, welches eingerichtet ist, um die Lanzette in der Applikationsposition zu stoppen und die Weiterbewegung des Trägerelements vorübergehend zu verhindern. Weiterhin kann die Greifvorrichtung mindestens einen Gegengreifer aufweisen, welcher mit dem Stoppelement zusammenwirkt. Dieses Zusammenwirken kann derart erfolgen, dass die Lanzette zwischen dem Gegengreifer und dem Stoppelement formschlüssig gehaltert wird. Alternativ oder zusätzlich kann auch eine andere Art der Halterung erfolgen, beispielsweise eine kraftschlüssige Halterung. Besonders bevorzugt ist jedoch ein Formschluss, bei welchem, wie unten noch näher ausgeführt wird, ein Haltebereich der Lanzette zwischen dem Gegengreifer und dem Stoppelement formschlüssig aufgenommen ist. Diese Aufnahme erfolgt vorzugsweise derart, dass ein Spiel zum Ausgleich von Toleranzen bei der Herstellung und/oder dem Aufbringen der Lanzette auf das Trägerelement ausgeglichen werden kann.

Der Gegengreifer ist vorzugsweise vollständig oder teilweise getrennt von der Positionierungsvorrichtung ausgebildet, kann jedoch grundsätzlich zumindest teilweise mit der Positionierungsvorrichtung bauteilidentisch ausgebildet sein. Das Stoppelement und der Gegengreifer sind vorzugsweise, wie unten noch exemplarisch näher ausgeführt wird, in Transportrichtung nicht beweglich ausgestaltet oder nur unwesentlich beweglich ausgestaltet. Auch eine andere Ausgestaltung ist jedoch grundsätzlich möglich.

Die Greifvorrichtung, insbesondere der Gegengreifer und/oder die Positionierungsvorrichtung, können vorzugsweise mindestens eine Rampe aufweisen, insbesondere eine Rampe, welche dem Transportelement und beispielsweise dem Lanzettenband zuweist. Diese Rampe kann eingerichtet sein, um ein Einführen der Lanzette zwischen den Gegengreifer und das Stoppelement zu ermöglichen. Beispielsweise können der Gegengreifer, das Positionierungselement und das Stoppelement auf einer Seite des Trägerelements angeordnet sein und das oben beschriebene optionale Greiferunterteil auf einer gegenüberliegenden Seite des Trägerelements, sodass das Trägerelement mit den Lanzetten zwischen dem Greiferunterteil und dem Stoppelement, dem Positionierungselement und dem Gegengreifer hindurch geführt wird. Der Gegengreifer kann derart relativ zum Stoppelement positioniert sein, dass die Lanzette bei einem Transport des Trägerelements in einer Transportrichtung, zum Beispiel einer Spulrichtung eines Trägerbands im Normalbetrieb, zunächst den Gegengreifer passiert und dann das Stoppelement erreicht, um an diesem anzuschlagen. Bei Passieren des Gegengreifers und/oder des Positionierungselements, beispielsweise des Niederhalters, kann die Rampe bewirken, dass eine Lanzette leichter unter dem Gegengreifer bzw. dem Positionierungselement hindurchgleitet, um in einen Zwischenraum zwischen dem Gegengreifer bzw. dem Positionierungselement und dem Stoppelement zu gelangen. Die Rampe kann auch eingerichtet sein, um den Gegengreifer zumindest leicht anzuheben.

Die Greifvorrichtung und/oder die Lanzettenvorrichtung können insbesondere derart ausgestaltet sein, dass die Lanzette einen von der Greifvorrichtung erfassbaren Haltebereich umfasst. Beispielsweise kann dieser Haltebereich getrennt oder auch einstückig mit einem Lanzettenkörper ausgebildet sein, welcher räumlich getrennt von der Lanzettenspitze ausgestaltet sein soll. Der Haltebereich kann vorzugsweise mindestens eine äußere Kontur aufweisen, die eine Beaufschlagung der Lanzette mit einer Kraft parallel zur Stechbewegung ermöglicht. Beispielsweise kann diese Kontur mindestens eine Einschnürung und/oder mindestens eine Verdickung aufweisen, sodass auch ohne einen Kraftschluss, vorzugsweise ohne Reibungskraft, eine Schubbewegung oder eine Zugbewegung durch die Greifvorrichtung auf die Lanzette übertragen werden kann. Das Stoppelement und vorzugsweise der Gegengreifer können eine der äußeren Kontur des Haltebereichs zumindest näherungsweise angepasste Innenkontur aufweisen. So kann beispielsweise zwischen dem Gegengreifer und dem Stoppelement ein Zwischenraum ausgestaltet sein, der in seiner Innenkontur im Wesentlichen der äußeren Kontur des Haltebereichs zumindest näherungsweise entspricht. Dabei können auch Toleranzen ausgeglichen werden, beispielsweise indem die Innenkontur geringfügig größer ausgestaltet ist als die äußere Kontur des Haltebereichs, sodass beispielsweise Positionierungstoleranzen und/oder Orientierungstoleranzen, die zum Beispiel bei der Herstellung und/oder Aufbringen der Lanzetten entstehen können, ausgeglichen werden können.

Die Greifvorrichtung kann insbesondere derart ausgestaltet sein, dass diese die Lanzette bzw. deren Haltebereich von mehreren Seiten her umschließt, also beispielsweise beidseitig in Längserstreckungsrichtung des Trägerelements oder beidseitig senkrecht zur Stechebene. Die Greifvorrichtung kann dementsprechend insbesondere derart ausgestaltet sein, dass diese während der Stechbewegung ein Verkippen der Lanzette verhindert.

Die Lanzette kann insbesondere derart ausgestaltet sein, dass diese über eine vergleichsweise lange Lanzettenspitze verfügt, die über den Haltebereich in Stechrichtung hinausragt. Insbesondere kann diese Lanzettenspitze einen spitz oder sich verjüngend zulaufenden Bereich aufweisen. Die Lanzettenspitze kann insbesondere derart lang ausgestaltet werden, dass diese durch eine Gehäuseöffnung eines Gehäuses der Lanzettenvorrichtung hindurchragt und eine ausreichende Einstichtiefe in ein Körpergewebe erzielen kann. Insbesondere kann die Lanzettenspitze eine Länge von mehr als 2,2 mm aufweisen.

Zur Stabilisierung der Lanzettengeometrie kann die Lanzette also einen Lanzettenkörper aufweisen, welcher im Bereich der Lanzettenspitze und/oder zum vorderen Ende in Stechrichtung hin spitz zuläuft. So kann das Lanzettenband insbesondere neben dem Trägerband und den Lanzettenelementen weitere Elemente umfassen, welche beispielsweise als Sterilschutz für die Lanzetten wirken. Dieser Sterilschutz kann beispielsweise in Form eines weiteren Bandes ausgestaltet sein, welches die Lanzetten einzeln oder insgesamt abdeckt. Alternativ oder zusätzlich kann der Sterilschutz auch individuelle Abdeckungen der Lanzetten umfassen, beispielsweise Taschen oder ähnliches. Der Sterilschutz kann beispielsweise während oder vor einem Stechvorgang entfernt, durchschnitten, durchstochen, abgehoben oder aufsonstige Weise ganz oder teilweise entfernt werden. Die Lanzettengeometrie kann insbesondere derart ausgestaltet sein, insbesondere durch die oben beschriebene Stabilisierung, dass beim Entfernen des Sterilschutzes eine ausreichende Stabilität besteht. Insbesondere kann eine derartige Form der Lanzette, bei der Lanzettenkörper in Stechrichtung, beispielsweise ausgehend von dem Haltebereich, spitz oder verjüngend zuläuft, bei Lanzetten von Vorteil sein, deren Lanzettenspitze eine Länge von mehr als 2,2 mm aufweist. Beispielsweise kann der sich verjüngende oder spitz zulaufende Teil des Lanzettenkörpers während des Stechvorgangs durch eine Austrittsöffnung der Lanzettenvorrichtung, beispielsweise eines Gehäuses des Lanzettenvorrichtung, austreten, so dass für den Stechvorgang die Länge der Lanzettenspitze vollständig genutzt werden kann. Alternativ oder zusätzlich ist es auch denkbar, dass die Wandstärke des Gehäuses im Bereich der Austrittsöffnung so stark reduziert wird, dass eine Länge der Lanzettenspitze und/oder des sich verjüngenden oder spitz zulaufenden Teils der Lanzette von mehr als 2,2 mm im Wesentlichen nicht erforderlich ist. Allgemein sei jedoch darauf hingewiesen, dass auch andere Geometrien der Lanzette verwendet werden können, so dass die vorliegende Erfindung allgemein nicht auf eine bestimmte Lanzettengeometrie beschränkt ist.

Die Greifvorrichtung kann neben den bereits beschriebenen Elementen weitere Elemente umfassen. So kann die Greifvorrichtung beispielsweise mindestens eine ortsfeste, das heißt beim Stechvorgang zumindest teilweise, vorzugsweise vollständig, nicht mitbewegte Greiferführung aufweisen. Zumindest das Stoppelement und vorzugsweise auch das Positionierungselement und vorzugsweise auch der Gegengreifer können beweglich in der Greiferführung gelagert sein. Beispielsweise kann die Greiferführung eine oder mehrere Schienen umfassen, in welchen das Stoppelement und/oder das Positionierungselement und/oder der Gegengreifer gleitend gelagert sind, wobei die Schienen vorzugsweise in Stechrichtung orientiert sind, beispielsweise parallel zur Stechrichtung oder untere einer Abweichung von nicht mehr als 20°, vorzugsweise um nicht mehr als 10°, zur parallelen Richtung. Die Greiferführung kann beispielsweise auf einer Grundplatte und/oder in einem Gehäuse der Lanzettenvorrichtung montiert sein und/oder Bestandteil der genannten Elemente sein.

Weiterhin kann die Greifvorrichtung mindestens eine Greiferfeder umfassen. Diese Greiferfeder kann eingerichtet sein, um das Stoppelement und das Positionierungselement und vorzugsweise auch den optionalen Gegengreifer mit einer Kraft in Richtung des Trägerelements zu beaufschlagen. Beispielsweise kann die Federkraft in Richtung senkrecht zur Stechebene wirken. Beispielsweise kann auf diese Weise erreicht werden, dass das Stoppelement in einer Ruheposition mit seinem mindestens einen optionalen Anschlag derart positioniert ist, dass eine ankommende Lanzette in der Applikationsposition gegen diesen Anschlag stößt. Zum Lösen des Stoppelements kann beispielsweise diese Federkraft der mindestens einen Greiferfeder überwunden werden. Die Greiferfeder kann beispielsweise mindestens eine Blattfeder umfassen. Beispielsweise können jeweils eine Blattfeder für das Stoppelement und für das Positionierungselement und optional auch für den Gegengreifer vorgesehen sein. Die Greiferfeder kann jedoch auch ganz oder teilweise integriert sein in eines oder mehrere dieser Elemente und/oder die genannten Elemente können ganz oder teilweise selbst als federnde Elemente ausgestaltet sein, wie unten noch näher noch ausgeführt wird. So kann insbesondere die Greiferfeder ganz oder teilweise in das Stoppelement und/oder das Positionierungselement und/oder den Gegengreifer integriert sein. Beispielsweise können das Stoppelement und das Positionierungselement und vorzugsweise auch der Gegengreifer zumindest teilweise als Federbauteile ausgestaltet sein. Beispielsweise können zu diesem Zweck verschiedene Federbleche und/oder Teile ein und desselben Federblechs verwendet werden. So können beispielsweise das Stoppelement, das Positionierungselement und der Gegengreifer ganz oder teilweise aus einem Federblech herausgearbeitet sein. Für dieses Herausarbeiten bieten sich beispielsweise Ätzprozesse und/oder Schneideprozesse an, beispielsweise ein Laserschneideprozess. Beispielsweise können die Federbauteile, insbesondere das Federblech, ganz oder teilweise parallel zu dem oben beschriebenen Greiferunterteil angeordnet sein, beispielsweise einer ebenen Auflagefläche des Greiferunterteils. Insbesondere können diese Federbauteile zumindest nahezu auf dem Greiferunterteil aufliegen, sodass das Trägerelement zwischen den Federbauteilen und dem Greiferunterteil geführt werden kann.

Das Stoppelement und das Positionierungselement und vorzugsweise der Gegengreifer können grundsätzlich aus einem starren Vollmaterial gefertigt sein. Alternativ oder zusätzlich können jedoch auch eines, einige oder alle dieser Elemente zumindest teilweise als Zungen eines Federblechs ausgestaltet sein. Unter einem Federblech ist dabei allgemein ein metallisches Bauteil zu verstehen, welches in Blechform vorliegt, also mit einer Dicke, welche erheblich geringer ist als seine maximale laterale Ausdehnung, beispielsweise um mindestens einen Faktor 10, vorzugsweise um mindestens einen Faktor 100 und besonders bevorzugt mehr. Das Federblech kann insbesondere zumindest teilweise elastische Eigenschaften aufweisen.

Alternativ oder zusätzlich kann auch mindestens ein Kunststoffmaterial für die Herstellung der Greifvorrichtung verwendet werden. So können insbesondere eines oder mehrere der Elemente Greiferfeder, Stoppelement, Positionierungselement und Gegengreifer, alternativ oder zusätzlich zu einer vollständigen oder teilweisen Herstellung aus einem metallischen Material, ganz oder teilweise aus einem Kunststoffmaterial hergestellt sein. Insbesondere die mindestens eine Greiferfeder kann ganz oder teilweise aus mindestens einem Kunststoffmaterial mit elastischen Eigenschaften hergestellt sein, wobei diese mindestens eine Greiferfeder auch wiederum ganz oder teilweise mit einem oder mehreren weiteren Elementen der Greifvorrichtung zusammengefasst sein kann.

Das Federblech kann eine als Stoppelement wirkende erste Zunge aufweisen, eine als das Positionierungselement, insbesondere der Niederhalter, wirkende zweite Zunge und vorzugsweise eine der ersten Zunge gegenüberliegende und als der Gegengreifer wirkende dritte Zunge aufweisen. Diese Zungen können vorzugsweise in derselben Ebene angeordnet sein. Die zweite Zunge, welche als das Positionierungselement wirkt, kann beispielsweise zumindest im Wesentlichen die Form des Haltebereichs der Lanzette aufweisen. Die als Stoppelement wirkende erste Zunge und die Gegengreifer wirkende dritte Zunge können, wie oben beschrieben, zwischen sich einen Zwischenraum aufweisen, welcher beispielsweise ganz oder teilweise durch die zweite Zunge ausgefüllt sein kann, welche bei Eintreten einer Lanzette in den Zwischenraum aus der gemeinsamen Ebene des Federblechs heraus gebogen werden kann, um die Lanzette aufzunehmen und diese mit einer Kraft zu beaufschlagen. Die zweite Zunge und/oder die optionale dritte Zunge können, wie oben dargestellt, mindestens eine Rampe aufweisen, um ein Einführen einer Lanzette in den Zwischenraum zwischen der ersten Zunge und der dritten Zunge zu erleichtern.

Das Federblech kann als ebenes oder gebogenes Federblech ausgestaltet sein. Zumindest im Bereich der Applikationsposition sollte das Federblech vorzugsweise eben ausgestaltet sein. Ein gebogenes Federblech kann beispielsweise ein U-Federblech umfassen.

Weitere bevorzugte Ausgestaltungen der Erfindung betreffen insbesondere die Lanzettenvorrichtung. So kann die Lanzettenvorrichtung weiterhin mindestens einen Stechantrieb umfassen. Dieser Stechantrieb soll eingerichtet sein, um den Greifer zu der Stechbewegung mit der Lanzette anzutreiben. Für mögliche Ausgestaltungen des Stechantriebs kann insbesondere auf die bereits oben zitierte WO2009/037341A1 verwiesen werden. Die dort dargestellte Antriebseinheit kann grundsätzlich auch im Rahmen der vorliegenden Erfindung als Stechantrieb oder als Teil des Stechantriebs verwendet werden. Auch andere Ausgestaltungen sind jedoch grundsätzlich möglich.

Wie oben dargestellt, kann das Stoppelement insbesondere als lösbares Stoppelement ausgestaltet sein. Ist also ein Stechantrieb vorgesehen, welcher eingerichtet ist, um die Greifvorrichtung zu der Stechbewegung anzutreiben, so kann dieser Stechantrieb vorzugsweise weiterhin eingerichtet sein, um das Stoppelement nach der Stechbewegung wieder zu lösen und die zuvor für die Stechbewegung verwendete Lanzette wieder freizugeben. Wie oben dargestellt, kann dies insbesondere durch eine Bewegung eines Anschlags des Stoppelements erfolgen, beispielsweise eine Bewegung mit zumindest einer Bewegungskomponente senkrecht zur Stechebene.

Diese Lösung des Stoppelements nach der Stechbewegung, welche vorzugsweise durch den Stechantrieb erfolgt, kann auf verschiedenen Weisen erfolgen. So kann der Stechantrieb beispielsweise mindestens ein sich bewegendes Element aufweisen. Weiterhin kann der Stechantrieb mindestens eine Erhöhung aufweisen, insbesondere eine Rampe und/oder eine Stufe, wobei die Erhöhung beispielsweise mit dem sich bewegenden Element des Stechantriebs verbunden sein kann. Diese Rampe und/oder Stufe kann auch beispielsweise und/oder Kulisse ausgebildet sein und/oder als eine andere Art von Erhöhung, mittels derer ein Lösen des Stoppelements möglich ist. Das sich bewegende Element des Stechantriebs kann beispielsweise ein sich linear bewegendes Element und/oder ein rotierendes Element umfassen. Die Erhöhung soll eingerichtet sein, um mindestens einen Anschlag des Stoppelements der Greifvorrichtung quer zur einer Längserstreckungsrichtung des Trägerelements, beispielsweise quer zur Stechebene, zu bewegen, beispielsweise anzuheben, um das Stoppelement zu lösen. Beispielsweise kann die Greifvorrichtung derart ausgestaltet sein, dass nach dem Stechvorgang während eines erneuten Spannens eines Energiespeichers des Stechantriebs, beispielsweise einer Stechfeder, über mindestens eine in dem Stechantrieb ausgebildete Erhöhung, beispielsweise eine als Treppe ausgebildete Kulisse, die Greifvorrichtung, insbesondere das Stoppelement, einseitig angehoben wird, so dass die Lanzette freigegeben wird und ein Transport des Lanzettenbands bzw. Trägerbands erfolgen kann. Das Positionierungselement, insbesondere der Niederhalter, kann das Lösen der Lanzette von der Greifvorrichtung in Transportrichtung erleichtern. Während des Transport des Trägerbands kann dabei allgemein die Greifvorrichtung ganz oder teilweise federgelagert auf dem Lanzettenband bzw. dem Trägerband anliegen. Die hierbei entstehende Reibung kann dabei minimiert werden, so dass der Transport des Trägerbands bzw. des Lanzettenbands im Wesentlichen nicht behindert wird.

Beispielsweise kann der Stechantrieb als sich bewegendes Element mindestens eine Antriebsscheibe aufweisen. Eine Achse dieser Antriebsscheibe kann vorzugsweise quer, insbesondere zumindest näherungsweise senkrecht zu einer Transportrichtung des Trägerelements im Bereich der Applikationsposition angeordnet sein. Beispielsweise kann diese Achse im Wesentlichen senkrecht zur Stechebene angeordnet sein oder im Wesentlichen in der Stechebene oder parallel zu der Stechebene angeordnet sein. Unter "im Wesentlichen" sind hierbei wiederum, wie bei allen Winkelangaben im Rahmen der vorliegenden Erfindung, auch Abweichungen von vorzugsweise nicht mehr als 20° tolerierbar, insbesondere von nicht mehr als 10° und besonders bevorzugt von nicht mehr als 5° und besonders bevorzugt Null. Ist eine derartige Antriebsscheibe vorgesehen, so kann beispielsweise diese Antriebsscheibe an ihrer Umfangsseite und/oder auf ihrer Flachseite angeordnet die genannte Erhöhung, beispielsweise die Rampe und/oder die Stufe, aufweisen, welche eingerichtet ist, um das Stoppelement zu lösen. So kann beispielsweise durch diese mindestens eine Erhöhung der genannte Anschlag mit mindestens einer Bewegungskomponente senkrecht zur Stechebene bewegt, beispielsweise angehoben, werden, um die Lanzette freizugeben. Alternativ oder zusätzlich zu einer Antriebsscheibe sind jedoch auch andere sich bewegende Elemente in dem Stechantrieb möglich. So können beispielsweise Antriebsstangen oder andere Arten von Antrieben alternativ oder zusätzlich eingesetzt werden. Auch derartige bewegliche Elemente können mit einer oder mehreren entsprechenden Erhöhungen zur Lösung des Stoppelements ausgestaltet werden.

Die vorgeschlagene Greifvorrichtung und die Lanzettenvorrichtung in einer oder mehreren der beschriebenen Ausgestaltungen weisen gegenüber bekannten Vorrichtungen dieser Art zahlreiche Vorteile auf. So lässt sich insbesondere eine Bauraum sparende Greifvorrichtung realisieren, welche leicht auch in kompakte Lanzettenvorrichtungen integrierbar ist. Auch eine Integration in kombinierte Geräte, beispielsweise Handgeräte, ist möglich, also in Geräte, welche neben der Lanzettenfunktion mindestens eine Analysefunktion aufweisen.

Weiterhin ist die vorgeschlagene Greifvorrichtung gut geeignet, um herstellungsbedingte Toleranzen auszugleichen, beispielsweise Toleranzen, welche bei der Herstellung der Lanzetten und/oder der Verbindung der Lanzetten mit dem Trägerelement entstehen. Wird beispielsweise ein Bandmagazin mit einem Lanzettenband verwendet, so sinken hierdurch die Anforderungen an den Fertigungsprozess dieses Bandmagazins erheblich.

Weiterhin können in der Greifvorrichtung unterschiedliche Funktionen kombiniert realisiert werden. So können Federfunktion, Halte- und/oder Greiffunktion und Stoppfunktion integriert sein, wodurch weiter die Baugröße reduziert werden kann. Weiterhin kann eine optimierte Lanzettengeometrie in Zusammenarbeit mit dem Greifer realisiert werden. Auf diese Weise können zusätzlich Abweichungen in einem Herstellungsprozess ausgeglichen werden und eine höhere Systemsicherheit realisiert werden. Die beschriebene Greifvorrichtung kann auf einfache Weise mit dem Stechantrieb der Lanzettenvorrichtung zusammenwirken, wobei der Stechantrieb auch ganz oder teilweise in die Greifvorrichtung selbst integriert sein kann.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigen:
- Figur 1: eine perspektivische Darstellung eines ersten Ausführungsbeispiels einer Lanzettenvorrichtung mit einer Greifvorrichtung;
- Figur 2: die Lanzettenvorrichtung gemäß Figur 1 in einer Explosionsdarstel- lung;
- Figuren 3A und 3B: Detaildarstellungen der Greifvorrichtung der Lanzettenvorrichtung gemäß Figur 1,
- Figur 4: ein alternatives Ausführungsbeispiel einer Greifvorrichtung mit ei- nem Blechbauteil;
- Figur 5: eine Detaildarstellung eines Formschlusses zwischen der Greifvor- richtung gemäß Figur 4 und einer Lanzette;
- Figur 6: ein alternatives Ausführungsbeispiel mit einem gebogenen Blech- bauteil;
- Figuren 7A bis 11B: verschiedene Verfahrensschritte eines Stechvorgangs unter Verwen- dung der in Figur 4 dargestellten Lanzettenvorrichtung und Greifvor- richtung; und
- Figuren 12A bis 16B: verschiedene Verfahrensschritte eines Stechvorgangs unter Verwen- dung der in Figur 6 gezeigten Variante des Blechbauteils.

### Ausführungsbeispiele

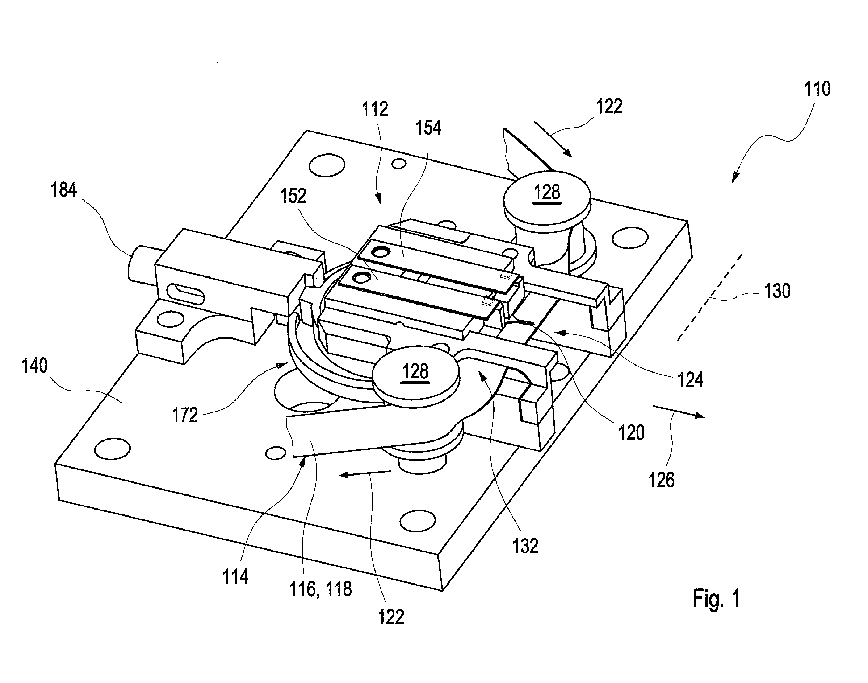

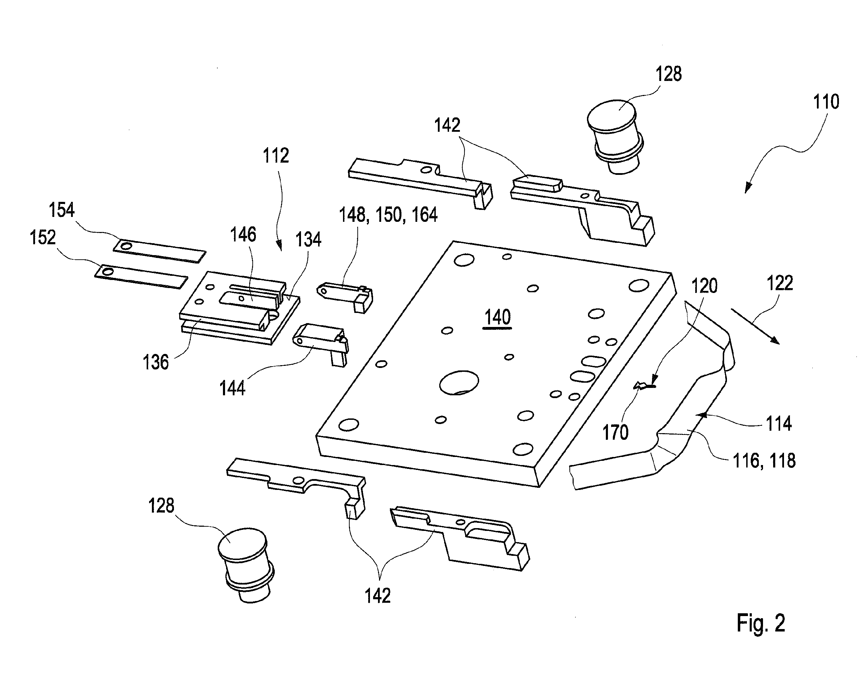

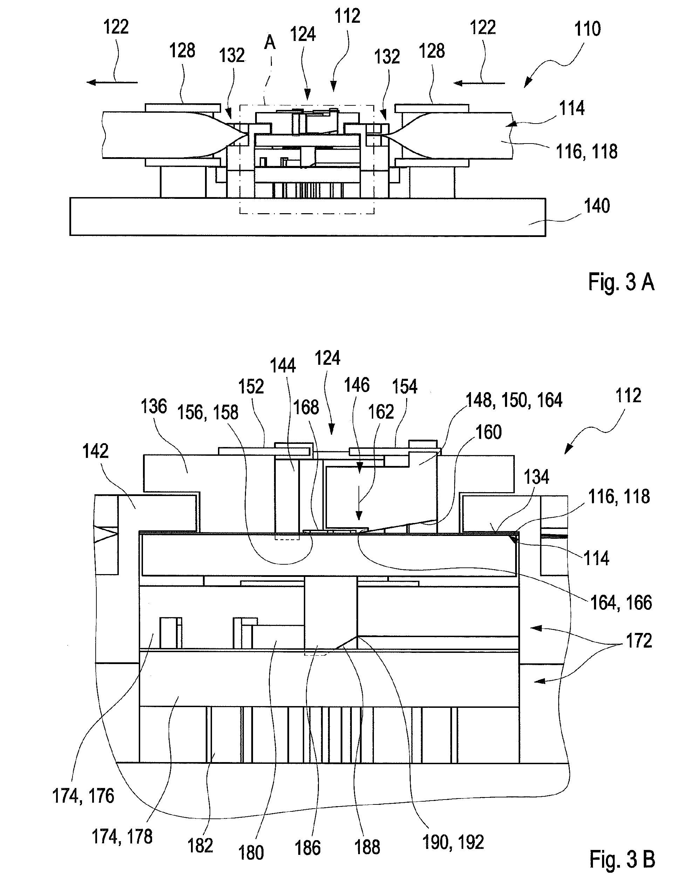

In den Figuren 1 bis 3B ist ein erstes Ausführungsbeispiel einer Lanzettenvorrichtung 110 mit einer Greifvorrichtung 112 dargestellt. Dabei zeigt Figur 1 eine perspektivische Darstellung von Teilen der Lanzettenvorrichtung 110. Figur 2 zeigt eine Explosionsdarstellung von Teilen der Lanzettenvorrichtung 110 gemäß Figur 1, insbesondere von Teilen der Greifvorrichtung 112. Figur 3A zeigt eine Vorderansicht der Lanzettenvorrichtung 110 mit Blick auf die Greifvorrichtung 112, und Figur 3B zeigt eine vergrößerte Darstellung des in Figur 3A umrandet dargestellten und mit A bezeichneten Bereichs der Greifvorrichtung 112. Sämtliche Figuren 1 bis 3B sollen im Folgenden gemeinsam erläutert werden. Es wird darauf hingewiesen, dass die Lanzettenvorrichtung 110 zahlreiche weitere Elemente enthalten kann, welche in den Figuren 1 bis 3B nicht dargestellt sind. So kann insbesondere weiterhin ein Gehäuse vorgesehen sein, welches alle oder einige der in den Figuren 1 bis 3B dargestellten Bauteile umschließen kann.

Die Lanzettenvorrichtung 110 umfasst in dem dargestellten Ausführungsbeispiel ein Lanzettenband 114. Diese Lanzettenband 114 umfasst seinerseits ein Trägerelement 116 in Form eines Trägerbands 118, auf welches eine Mehrzahl von Lanzetten 120 aufgebracht ist. Unter "Aufbringen" ist dabei, neben der Möglichkeit auf eine Oberfläche des Trägerbands 118, auf die Möglichkeit zu verstehen, dass die Lanzetten 120 eingebettet sind zwischen mehrere Elemente des Trägerelements 116, beispielsweise zwischen mehrere Schichten. Das Trägerband 118 kann beispielsweise als Kunststoff und/oder Papierband ausgestaltet sein. Das Trägergand 118 kann beispielsweise von einem in den Figuren nicht dargestellten Gutwickel bereitgestellt werden, in einer Spulrichtung 122 durch die Lanzettenvorrichtung 110 gespult werden und schließlich auf einen ebenfalls nicht dargestellten Schlechtwickel aufgewickelt werden. Zum Zweck des Spulens kann beispielsweise der Schlechtwickel angetrieben werden.

Mittels dieses Spulmechanismus werden die Lanzetten 120 nacheinander in einer Applikationsposition 124 der Lanzettenvorrichtung 110 bereitgestellt, dort von der Greifvorrichtung 112 ergriffen und für eine Stechbewegung in einer in Figur 1 mit der Bezugsziffer 126 bezeichneten Stechrichtung eingesetzt. Zu diesem Zweck wird das Trägerband 118 vorzugsweise vor und nach der Umlenkposition 124 mittels Umlenkrollen 128 umgelenkt, sodass dieses zumindest im Bereich der Applikationsposition 124 eine in Figur 1 mit der Bezugsziffer 130 bezeichnete und symbolisch dargestellte Längserstreckungsrichtung aufweist, welche vorzugsweise im Wesentlichen senkrecht zur Stechrichtung 126 verläuft. Wie oben dargestellt, sind jedoch auch leichte Abweichungen von dieser Senkrechten möglich. Die Längserstreckungsrichtung 130 und die Stechrichtung 126 definieren gemeinsam eine Stechebene. Mittels einer Bandumlenkung 132 kann das Trägerband 118 im Bereich der Applikationsposition 124 derart umgelenkt bzw. umgebogen werden, dass dieses Teile desselben im Bereich der Applikationsposition 124 im Wesentlichen parallel zu dieser Stechebene verläuft. Dies ist besonders gut erkennbar in der Explosionsdarstellung gemäß Figur 2 oder in der Vorderansicht gemäß Figur 3A. Während des Spulvorgangs verläuft das Lanzettenband 114 auf einer Auflagefläche 134 eines Greiferunterteils 136. Dies ist besonders deutlich erkennbar in den Darstellungen gemäß den Figuren 2 und 3B. Das Greiferunterteil 136, welches einteilig oder auch mehrteilig ausgestaltet sein kann, weist eine Führungsnut138 auf, in welcher in diesem Ausführungsbeispiel eine ortsfest zu einer Trägerplatte 140 oder einem Gehäuse oder Gehäuseteil der Lanzettenvorrichtung 110 gelagerte Greiferführung 142 (siehe ebenfalls die Detaildarstellung in 3B) aufgenommen ist. Auf diese Weise ist mittels der Greiferführung 142 die Greifvorrichtung 112 oder ein Teil derselben in Stechrichtung 126 beweglich gelagert und kann die Stechbewegung durchführen.

Die Greifvorrichtung 112 weist in dem dargestellten Ausführungsbeispiel einen mehrteiligen Aufbau auf, welcher anhand der Detaildarstellung in Figur 3B erläutert werden soll. So weist die Greifvorrichtung 112 ein Stoppelement 144 auf, welches in einer Aufnahme 146 des Greiferunterteils 136 derart schwenkbar gelagert ist, dass dieses eine Schwenkbewegung mit seinem am weitesten in Stechrichtung 126 ragenden Teil um eine Schwenkachse durchführen kann, welche beispielsweise in der Stechebene angeordnet ist. Ähnlicherweise ist in der Aufnahme 146 ein Positionierungselement 148 in Form eines Niederhalters 150 schwenkbar gelagert, beispielsweise um dieselbe Schwenkachse wie das Stoppelement 144. Das Stoppelement 144 und der Niederhalter 150 sind mit separaten Greiferfedern 152, 154 mit einer Federkraft senkrecht zur Stechebene, hin zum Lanzettenband 114, beaufschlagt.

Das Stoppelement 144 weist in dem dargestellten Ausführungsbeispiel einen Anschlag 156 in Form einer Anschlagskante 158 auf. Beim Spulen des Lanzettenbands 114 wird das Stoppelement 144 durch die Greiferfeder 152 nach unten gegen das Lanzettenband 114 gepresst, sodass eine in der Applikationsposition 124 angekommene Lanzette 120 (in Figur 3B nicht dargestellt) an dem Anschlag 156 anstößt. Ein weiteres Spulen des Lanzettenbands 114 wird hierdurch gestoppt. Beispielsweise kann ein Antrieb des Schlechtwickels derart ausgestaltet sein, dass dieser eine Rutschkupplung aufweist, welche aufgrund des erhöhten Drehmoment-Aufwands, wenn eine Lanzette 120 in der Applikationsposition 124 den Anschlag 156 erreicht, durchrutscht. Auf diese Weise kann ein Restlauf eines Lanzettenband-Antriebs fortgesetzt werden, ohne dass hierdurch das Lanzettenband 114 beschädigt wird.

Das Positionierungselement 148 in Form des Niederhalters 150 weist eine Rampe 160 zum Einführen des Lanzettenbands auf. Diese Rampe 160 bewirkt, dass beim Einführen der Lanzette 120 der Niederhalter 150 gegen die Kraft der Greiferfeder 154 angehoben wird, damit die Lanzette 120 zum Stoppelement 144 gelangen kann. In der Applikationsposition 124 wird dann die Lanzette 120 durch das Positionierungselement 148 mit einer Kraft 162 senkrecht zur Stechebene (welche in Figur 3B horizontal und senkrecht zur Zeichenebene verläuft) beaufschlagt und so in der Stechebene oder in einer Ebene parallel zur Stechebene gehalten.

Weiterhin kann die Greifvorrichtung 112, wie im Ausführungsbeispiel in den Figuren 1 bis 3B, einen Gegengreifer 164 aufweisen. Dieser Gegengreifer 164 ist in dem dargestellten Ausführungsbeispiel einteilig mit dem Positionierungselement 148 ausgestaltet, kann jedoch auch als separates Bauteil ausgestaltet sein. Die Rampe 160 kann entsprechend auch Bestandteil des Gegengreifers 164 sein. Der Gegengreifer 164 weist in dem dargestellten Ausführungsbeispiel eine der Anschlagskante 158 gegenüberliegende Kante 166 auf. Zwischen der Kante 166 und der Anschlagskante 158 ist ein Zwischenraum 168 ausgebildet, in welchem ein Haltebereich 170 der Lanzette 120 aufgenommen wird, wenn die Lanzette 120 in der Applikationsposition 124 ankommen ist. Der Haltebereich 170 kann eine Kontur aufweisen, welche - abgesehen von möglichen Toleranzen und einem Toleranzausgleich - der äußeren Kontur des Haltebereichs 170 entsprechen kann. Beispiele einer derartigen Kontur werden in folgenden Ausführungsbeispielen noch näher erläutert.

Die Lanzettenvorrichtung 110 weist weiterhin einen Stechantrieb 172. Dieser Stechantrieb 172 kann ganz oder teilweise gekoppelt sein mit einem Bandantrieb der Lanzettenvorrichtung 110. Eine exemplarische Ausgestaltung des Stechantriebs 172 soll anhand der Figur 3B erläutert werden.

So weist in dem dargestellten Ausführungsbeispiel der Stechantrieb 172 eine mehrteilig ausgebildete Antriebsscheibe 174 auf. Diese wiederum weist eine mit dem Greiferunterteil 136 gekoppelte Greiferantriebsscheibe 176 und eine unterhalb dieser Greiferantriebsscheibe 176 angeordnete Spannscheibe 178 auf. Die Greiferantriebsscheibe 176 und Spannscheibe 178 können über eine Stechfeder 180, beispielsweise in Form einer Spiralfeder, miteinander gekoppelt sein. Die Kopplung zwischen der Greiferantriebsscheibe 176 und dem Greiferunterteil 136 kann beispielsweise über einen in Figur 3B nicht erkennbaren Nocken an der Greiferantriebsscheibe 176 erfolgen, welcher in einer entsprechende Nut auf der Unterseite des Greiferunterteils 136 eingreift.

Während eines Spannvorgangs, währenddessen das Lanzettenband 114 weitergetaktet wird, wird die Spannscheibe 178 gedreht, beispielweise mittels eines Zahnrades 182 auf der Unterseite der Spannscheibe 178. Die Greiferantriebsscheibe 176 wird hierbei nicht oder nicht vollständig mit gedreht, sodass die Stechfeder 180 gespannt wird. Mittels eines Auslösemechanismus 184 wird die Greiferantriebsscheibe 176 im gespannten Zustand gehalten.

Gleichzeitig wird während dieser langsamen Bewegung der Spannscheibe 178 das Lanzettenband 114 weitergetaktet. Eine Lanzette 120 gleitet dabei unter der Rampe 160 hindurch, hebt kurzfristig den Niederhalter 150 an und wird schließlich am Anschlag 156 des Stoppelements 144 gestoppt. Der Niederhalter 150 bewegt sich wieder nach unten, sodass die Lanzette 120 in der Applikationsposition 124 in dem Zwischenraum 168 aufgenommen ist. Gleichzeitig wird, wie oben beschrieben, während dieses Vorgangs die Stechfeder 180 gespannt. Nach Auslösen des Auslösemechanismus 184 wird die Greiferantriebsscheibe 176 und/oder die Stechfeder 180 durch den Auslösemechanismus 184 freigegeben, und die Greiferantriebsscheibe 176 kann eine schnelle Rotationsbewegung durchführen, während derer das Greiferunterteil 136 mit dem Stoppelement 144 und dem Positionierungselement 148 sowie optional dem Gegengreifer 164 zu einer schnellen Stechbewegung in Stechrichtung 126 sowie einer anschließenden Rückwärtsbewegung angetrieben werden. Die Stechfeder 180 wird dabei entspannt. Nach diesem Stechvorgang kann dann ein Weitertakten des Lanzettenbands 114 in Spulrichtung 122 erfolgen. Zu diesem Zweck kann das Stoppelement 144 lösbar ausgestaltet sein, um einen Weitertransport des Lanzettenbands 114 zu ermöglich. In dem dargestellten Ausführungsbeispiel weist das Stoppelement 144 zu diesem Zweck einen Fortsatz 186 auf, welcher durch einen Durchgriff in der Greiferantriebsscheibe 176 hindurch nach unten ragt und eine Rampe 188 aufweist. Dieser Fortsatz 186 wirkt seinerseits mit einer Rampe 190 oder einer Stufe 192 auf der Spannscheibe 178 und/oder anderen Teilen der Antriebsscheibe 174 zusammen. Wird die Spannscheibe 178 beim Weitertakten gedreht, so wird durch diese Rampe 190 bzw. Stufe 192 mittels des Fortsatzes 186 das Stoppelement 144 angehoben, sodass die Lanzette 120 aus dem Zwischenraum 168 freigegeben wird und ein Weitertakten ermöglicht wird. Beispielsweise kann dieses Anheben des Stoppelements 144 durch die Rampe 190 bzw. Stufe 192 für einen Drehwinkel von maximal dreißig Grad der Spannscheibe 178 erfolgen. Dann endet die Stufe 192 bzw. die Rampe 190 wieder, und das Stoppelement 144 bewegt sich wieder nach unten, sodass der Greifer wieder schließt. Damit beim Anheben des Stoppelements 144 die Lanzette 120 nicht mit bewegt wird, kann die Lanzette während dieses Öffnungsvorgangs weiterhin mit dem Niederhalter 150 heruntergedrückt werden oder zumindest während einer Zeit dieses Anhebungsvorgangs. Sämtliche Teile des Greifers können von den Greiferfedem 152, 154, welche beispielsweise als Plattfedern ausgestaltet sein können, permanent angedrückt werden.

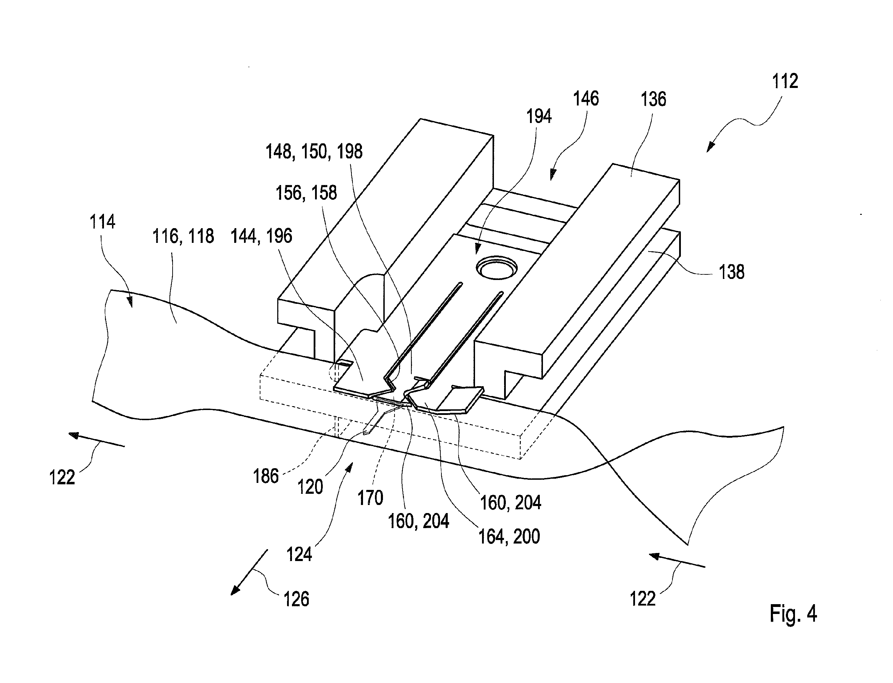

In dem Ausführungsbeispiel gemäß den Figuren 1 bis 3B besteht der eigentliche Greifer der Greifvorrichtung 112 somit aus dem Stoppelement 144, dem Positionierungselement 148 und optional dem Gegengreifer 164, sowie den zugehörigen Greiferfedern 152, 154. Die Greifer bildet die beweglichen Teile der Greifvorrichtung 112, welche das Ergreifen einer Lanzette 120 in Applikationsposition 124 ermöglichen. Diese mindestens 5-teilige Ausgestaltung des eigentlichen Greifers kann stark vereinfacht werden, indem diese Bauteile ganz oder teilweise zusammengefasst werden. So ist in Figur 4 ein alternatives Ausführungsbeispiel der Greifvorrichtung 112 gezeigt, bei welcher die genannten Teile des Greifers, zuzüglich des Greiferunterteils 136, auf zwei Teile reduziert werden können. So ist zunächst wieder ein Greiferunterteil 136 vorgesehen, welches ähnlich zum Greiferunterteil 136 in dem Ausführungsbeispiel gemäß den Figuren 1 bis 3B ausgestaltet sein kann und welches beispielsweise wiederum eine Führungsnut 138 aufweisen kann. In eine Aufnahme 146 dieses Greiferunterteils 136 ist jedoch, anstelle der verschiedenen einzelnen, oben genannten Elemente 144, 148, 164 sowie 152 und 154 ein einziges Greiferblech 194 oder mehrere derartiger Greiferbleche 194 integriert. Dieses Greiferblech ist als Federblech ausgestaltet und weist eine erste Zunge 196, eine zweite Zunge 198 und eine dritte Zunge 200 auf. Während die erste Zunge 196 einen Anschlag 156 aufweist und als Stoppelement 144 wirkt, wirkt die mittlere, zweite Zunge 198 als Positionierungselement 148 und Niederhalter 150. Die am weitesten entgegen der Spulrichtung 122 angeordnete dritte Zunge 200 weist ihrerseits eine der Anschlagskante 158 gegenüberliegende Kante 160 auf und wirkt als Gegengreifer 164. Das Greiferblech 194 kann beispielsweise über eine Fixierung 202 mit dem Greiferunterteil 136 verbunden sein. Die Federwirkung, welche ursprünglich durch die Greiferfedern 152, 154 ausgeübt wurde, kann nun aufgrund zumindest teilweise elastischer Eigenschaften des Greiferblechs 194, welches als Federblech ausgestaltet sein kann, in das Greiferblech 194 integriert sein.

Weiterhin kann das Greiferblech 194 gebogen sein und an seiner am weitesten in Spulrichtung 122 angeordneten Seite einen nach unten ragenden Fortsatz 186 aufweisen. Weiterhin kann auf der am weitesten entgegen der Spulrichtung 122 angeordneten Seite der Gegengreifer 164 eine Rampe 160 aufweisen, welche bei der Ausgestaltung als Greiferblech 194 besonders einfach in Form einer nach oben gebogenen Lasche 204 realisierbar ist. Die Ausgestaltung mittels des Greiferblechs 194 kann im Wesentlichen die gleiche Funktion wie bei dem in den Figuren 1 bis 3B beschriebenen Ausführungsbeispiel aufweisen. So kann bezüglich der Verfahrensabläufe des Weitertaktens einer Lanzette 120 weitgehend auf die obige Beschreibung verwiesen werden.

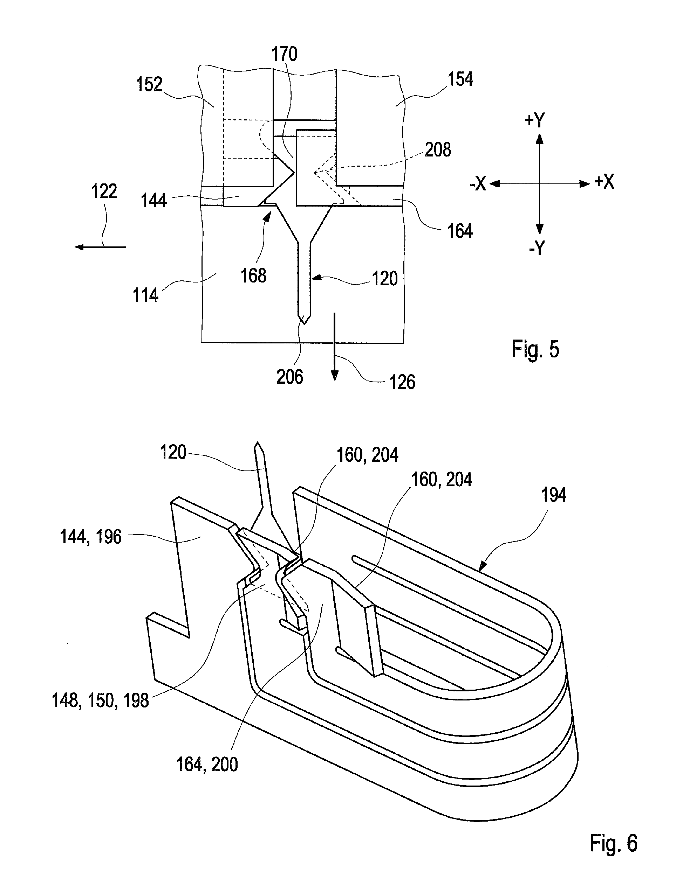

Die neue Geometrie der Greifvorrichtung 112 gemäß dem ersten oder auch dem zweiten Ausführungsbeispiel ermöglicht die Realisierung eines formschlüssigen Greifers, alternativ oder zusätzlich zu einem kraftschlüssigen Greifer. Dies ist in Figur 5 exemplarisch dargestellt. Figur 5 zeigt eine Lanzette 120 mit Darstellung in einer Stechebene. Die Lanzette 120 weist eine Lanzettenspitze 206 sowie einen Haltebereich 170 auf. Der Haltebereich 170 seinerseits weist eine Kontur auf, welche ein formschlüssiges Ergreifen der Lanzette 120 durch die Greifvorrichtung 112 ermöglicht. So weist der Haltebereich 170 eine Außenkontur mit einer Einschnürung 208 auf. Ein Zwischenraum 168 zwischen dem Stoppelement 144 und dem Gegengreifer 164 kann seinerseits eine Innenkontur aufweisen, welche, abgesehen von Positionierungstoleranzen und/oder Fertigungstoleranzen, im Wesentlichen der Außenkontur des Haltebereichs 170 entsprechen kann.

Die Längserstregungsrichtung des Lanzettenbands 114 definiert in der Darstellung gemäß Figur 5 eine x-Richtung, wohingegen die Stechrichtung 126 eine y-Richtung definiert. Die neue Lanzettengeometrie ermöglicht einen formschlüssigen Greifer, welcher sicherstellt, dass während des Stechvorgangs die Lanzette 120 weder in +/- -x-Richtung oder in +/- -y-Richtung ausweichen kann. Eine Bewegung in +/- -z-Richtung, welche als Richtung senkrecht zu der Stechebene definiert ist, wird durch den in Figur 5 nicht dargestellten Niederhalter 150 ausgeschlossen oder zumindest weitgehend vermindert. Es können in +/- -y-Richtung Toleranzen ausgeglichen werden, wie auch in +/- -x-Richtung, welche beispielsweise während eines Fertigungsprozesses der Lanzettenaufbringung entstehen können.

Das in Figur 4 dargestellte Greiferblech 194 ist lediglich exemplarisch dargestellt. So zeigt Figur 6 eine alternative Ausgestaltung des Greiferblechs 194, bei welchem dieses Greiferblech 194 U-förmig gebogen ausgestaltet ist. Ansonsten entspricht die Funktion des Greiferblechs 194 im Wesentlichen der oben anhand der Figur 4 beschriebenen Funktion.

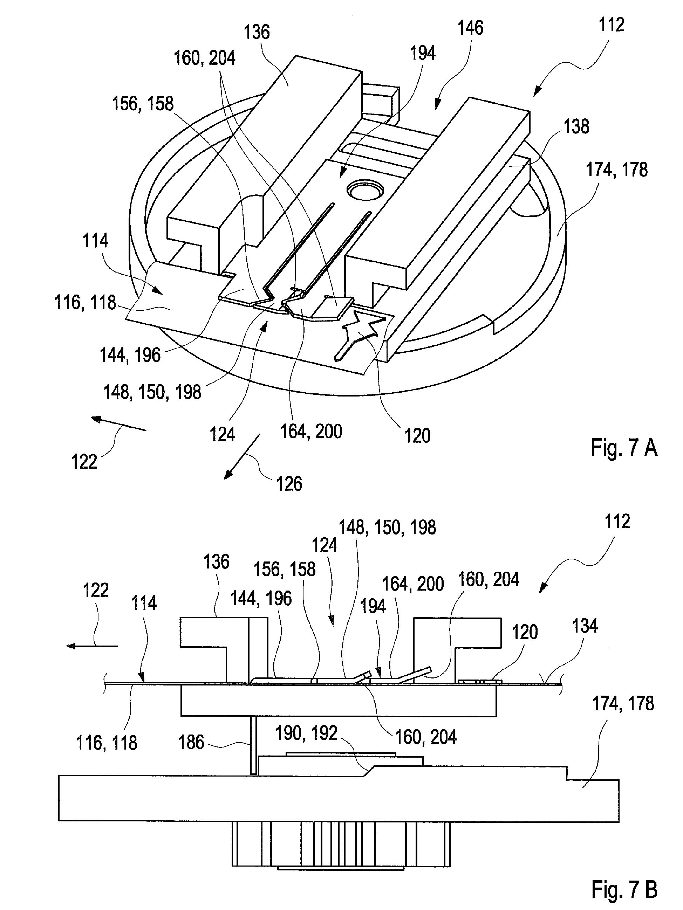

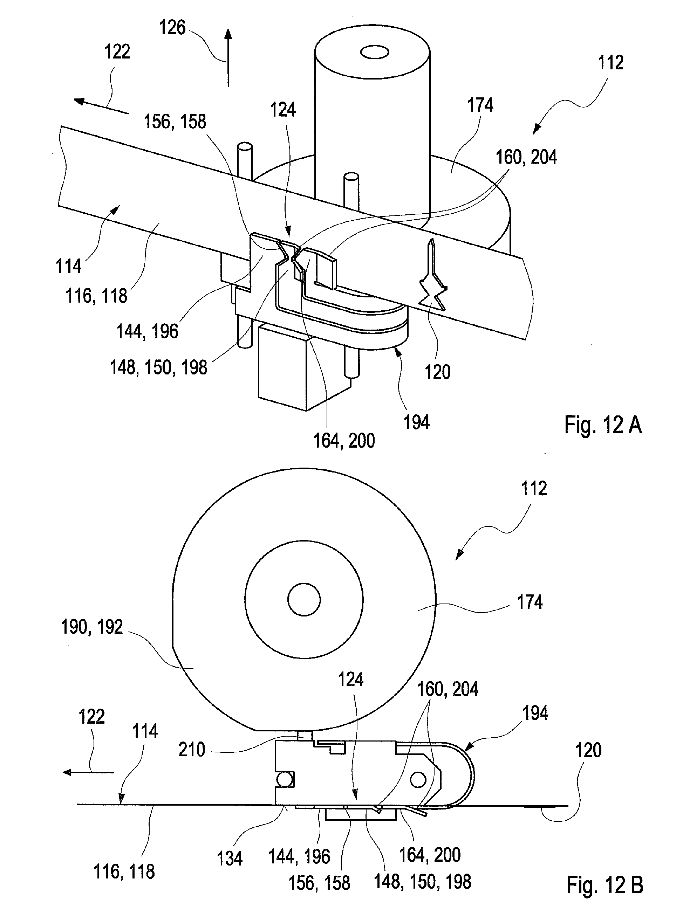

Die Funktion der Ausführungsbeispiele in den Figuren 4 und 6, bei welchen jeweils Greiferbleche 194 verwendet werden, soll anhand im Folgenden dargestellter Funktionsabläufe kurz erläutert werden. Dabei zeigen im Folgenden die Figuren 7A bis 11B einen Funktionsablauf des Ausführungsbeispiels gemäß Figur 4, wohingegen die Figuren 12A bis 16B einen Funktionsablauf des Ausführungsbeispiels gemäß Figur 6 darstellen. In den Zeichnungen zeigen jeweils die mit "A" bezeichneten Figuren eine perspektivische Darstellung, wohingegen die entsprechenden, mit "B" bezeichneten Figuren eine Forderansicht der Greifvorrichtung 112 oder von Teilen derselben zeigt, mit Zeichenebene senkrecht zur Stechebene. Für die Beschreibung der einzelnen Elemente kann im Wesentlichen auf die obigen Figuren 4 und 6 verwiesen werden.

Bei dem Ausführungsbeispiel in den Figuren 7A bis 11B ist die Stechrichtung 126 senkrecht zur Wickelachse des Gutwickels und/oder des Schlechtwickels und senkrecht zu einer Achse der Antriebsscheibe 174. Von dieser Antriebsscheibe 174 ist jeweils nur die Spannscheibe 178 gezeigt, die Greiferantriebsscheibe 176 ist zur Vereinfachung weggelassen.

In den Figuren 7A und 7B ist eine Situation dargestellt, bei welcher eine Lanzette 120 vor der Greifvorrichtung 112 ankommt. In der Frontalansicht gemäß Figur 7B ist die Rampe 160 deutlich zu erkennen, mit welcher beim Einführen die als Gegengreifer 164 wirkende dritte Zunge 200 geöffnet wird.

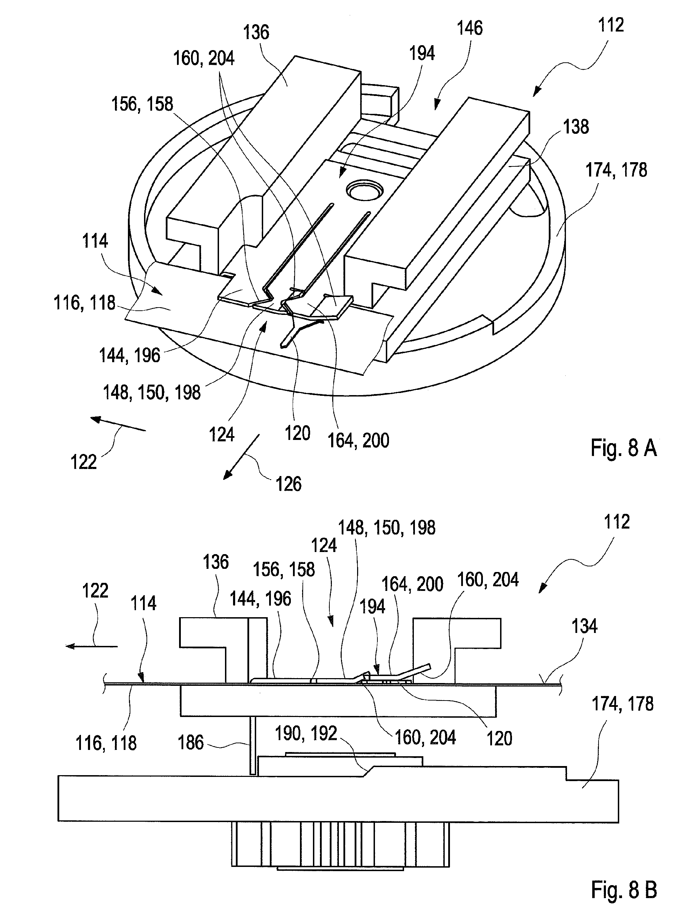

Dieser Öffnungsvorgang ist in den Figuren 8A und 8B dargestellt. In dieser Situation passiert die Lanzette 120 gerade den Gegengreifer 164 und hebt dabei die dritte Zunge 200 an. Um bei diesem Einführen ein Anheben der zweiten Zunge 198 zu erleichtern, kann in diesem und auch in anderen Ausführungsbeispielen alternativ oder zusätzlich auch das Positionierungselement 148, insbesondere der Niederhalter 150, eine entsprechende Rampe 160 aufweisen, beispielsweise ebenfalls in Form einer nach oben gebogenen Lasche 204.

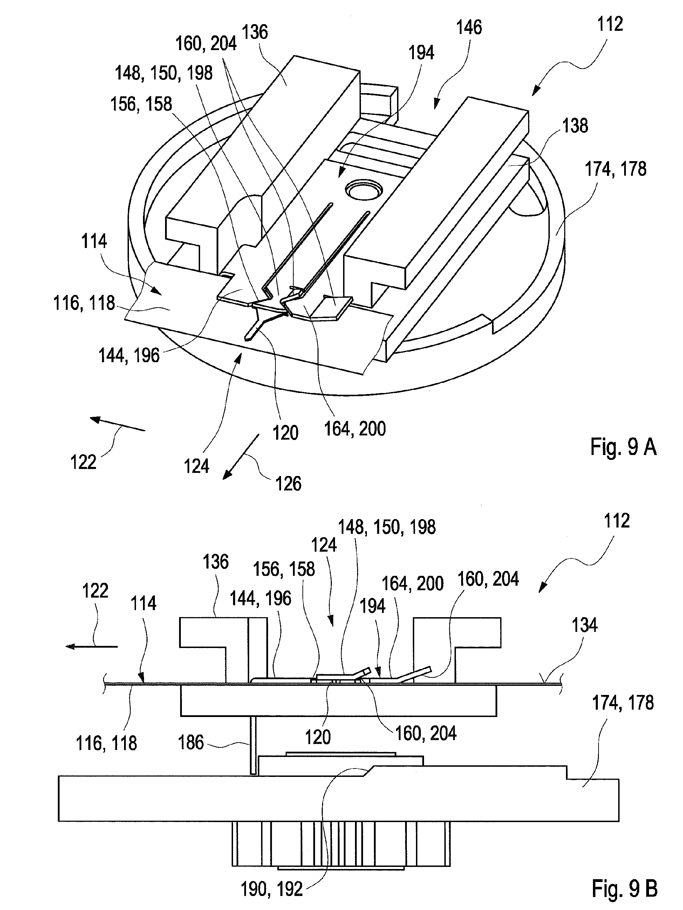

In den Figuren 9A und 9B ist schließlich eine Situation dargestellt, in welcher die Lanzette 120 in der Applikationsposition 124 angekommen ist. Die Lanzette 120 wird dabei an der Anschlagskante 158 des Stoppelements 144 gestoppt und ist damit in der Greiferstruktur gefangen. Die dritte Zunge 200 hat sich aufgrund der eigenen Federkraft wieder nach unten, gegen die Auflagefläche 134 bzw. das Trägerband 118 des Lanzettenbands 114 bewegt. Die Lanzette 120 kann sich nur noch in diesem Formschluss zwischen dem Stoppelement 144 und dem Gegengreifer 164 minimal bewegen.

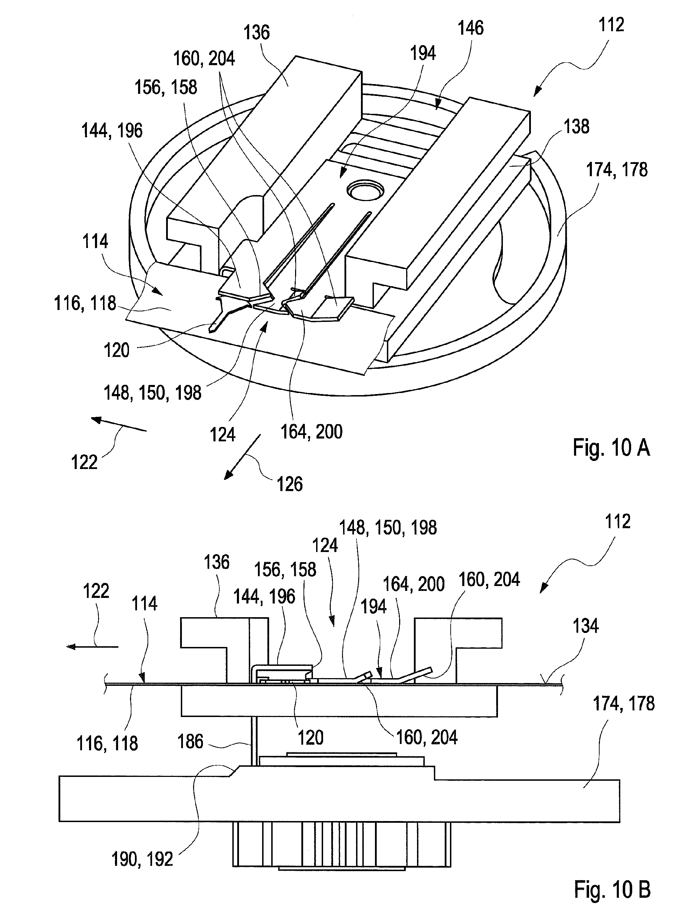

Anschließend wird der Stechvorgang durchgeführt, wie er oben beschrieben wurde. In den Figuren 10A und 10B ist schließlich eine Situation dargestellt, bei welcher, nach dem Stechvorgang, ein Weitertakten des Lanzettenbands 114 erfolgt. Mittels des als Öffner wirkenden Fortsatzes 186 und der Rampe 190 bzw. Stufe 192 in der Spannscheibe 178 wird die erste Zunge 196, also das Stoppelement 144, angehoben, während die Lanzette 120 durch den Niederhalter 150 gehalten werden kann. Dementsprechend kann die Lanzette 120 aus der Greifvorrichtung 112 herausgezogen werden.

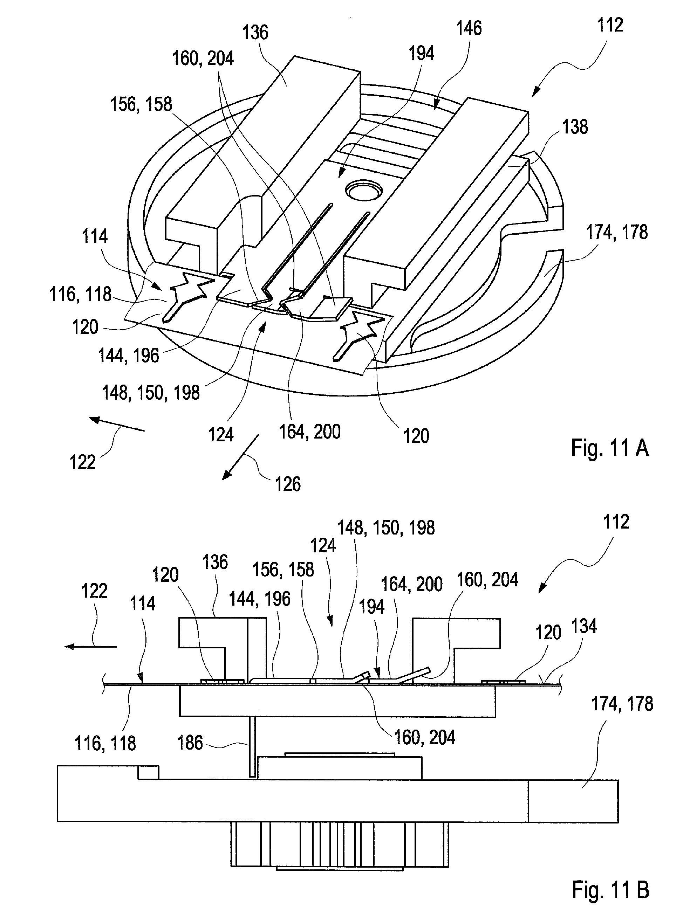

Die gebrauchte Lanzette 120 ist nun, wie in den Figuren 11A und 11B dargestellt, aus der Greifvorrichtung 112 befreit, und eine Lanzette 120, welche vorzugsweise ungebraucht ist, kann die in die Greifvorrichtung 112 transportiert werden. Die als Stopplasche wirkende erste Zunge 196 liegt in dieser Situation wieder auf dem Trägerband 118 auf.

In den Figuren 12A bis 16B ist ein den Figuren 7A bis 11B entsprechender Bewegungsablauf für das Ausführungsbeispiel der Greifvorrichtung 112 mit dem Greiferblech 194 gemäß Figur 6 dargestellt. Die Stechrichtung ist hierbei längs zu einer Wickelachse eines Gutwickels und/oder eines Schlechtwickels und/oder der Antriebsscheibe 174. Wie anhand der Figur 6 beschrieben, wirkt in diesem Ausführungsbeispiel die erste Zunge 196 des Greiferblechs 194 als Stoppelement 144 die zweite Zunge 198 als Positionierungselement 148 bzw. Niederhalter 150, und die dritte Zunge 200 als Gegengreifer 164. Die Antriebsscheibe 174 kann wiederum eine Rampe 190 und/oder eine Stufe 192 aufweisen, welche, beispielsweise über ein Übertragungselement 210 auf die erste Zunge 196 einwirken kann, um diese anzuheben.

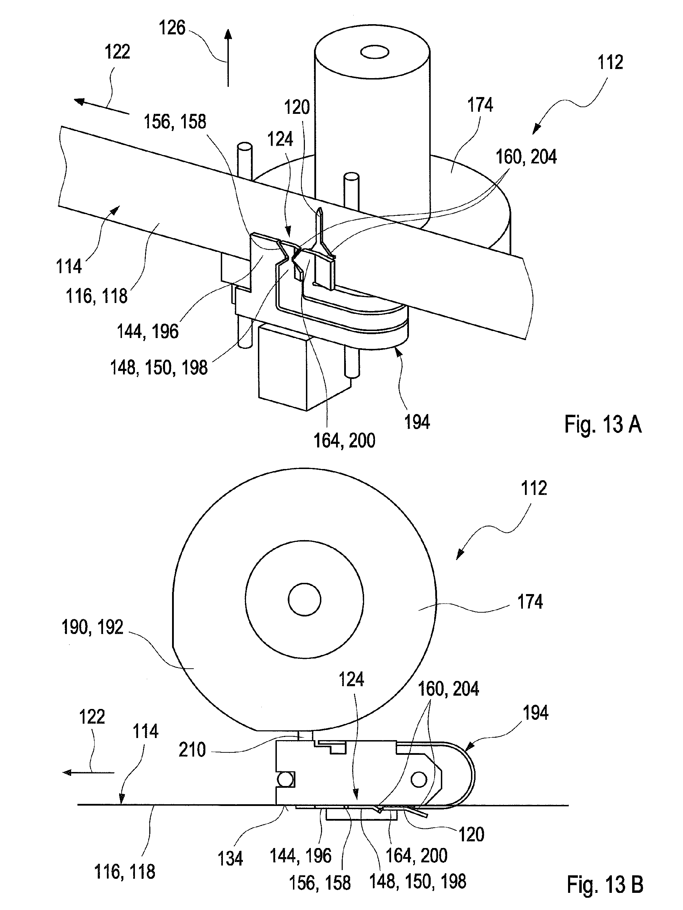

In den Figuren 12A und 12B ist wiederum eine Situation analog in den Figuren 7A und 7B dargestellt, bevor eine Lanzette 120 die Greifvorrichtung 112 erreicht. In den Figuren 13A und 13B ist eine Situation dargestellt, welche der Situation in den Figuren 8A und 8B entspricht. Die Lanzette 120 hat die Rampe 160 der dritten Zunge 200 erreicht und hebt diese an, um in die Greifvorrichtung 112 zu gelangen.

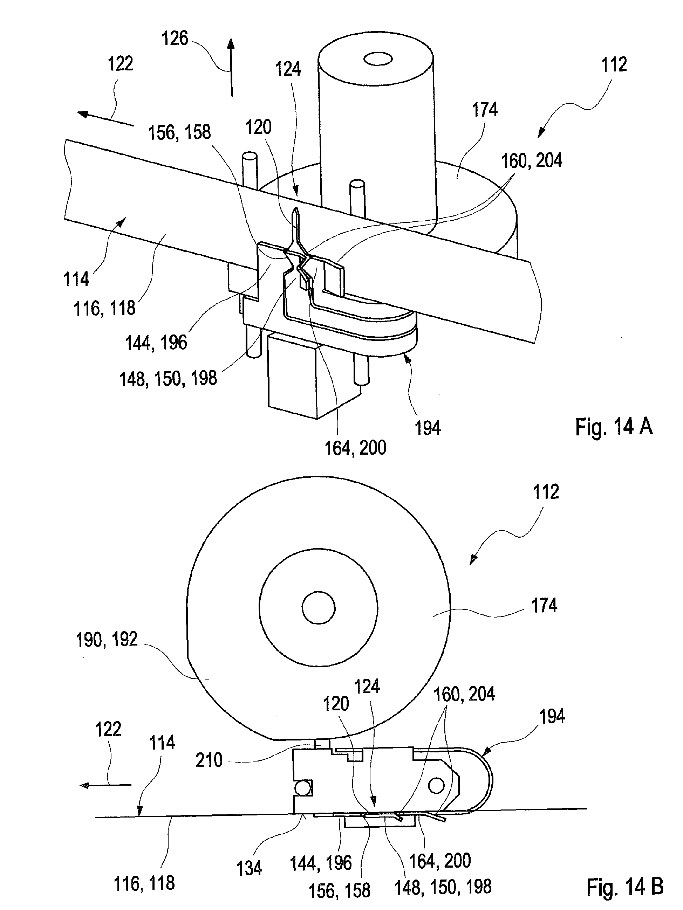

In den Figuren 14A und 14B ist eine Situation analog zur Situation in den Figuren 9A und 9B dargestellt, in welcher die Lanzette 120 in dem Zwischenraum 168 zwischen der ersten Zunge 196 angekommen ist und von dem Stoppelement 144 in Form der ersten Zunge 196 gestoppt ist. Der Niederhalter in Form der zweiten Zunge 198 ist in dieser Situation angehoben und drückt die Lanzette 120 nach unten, gegen eine Auflagefläche 134.

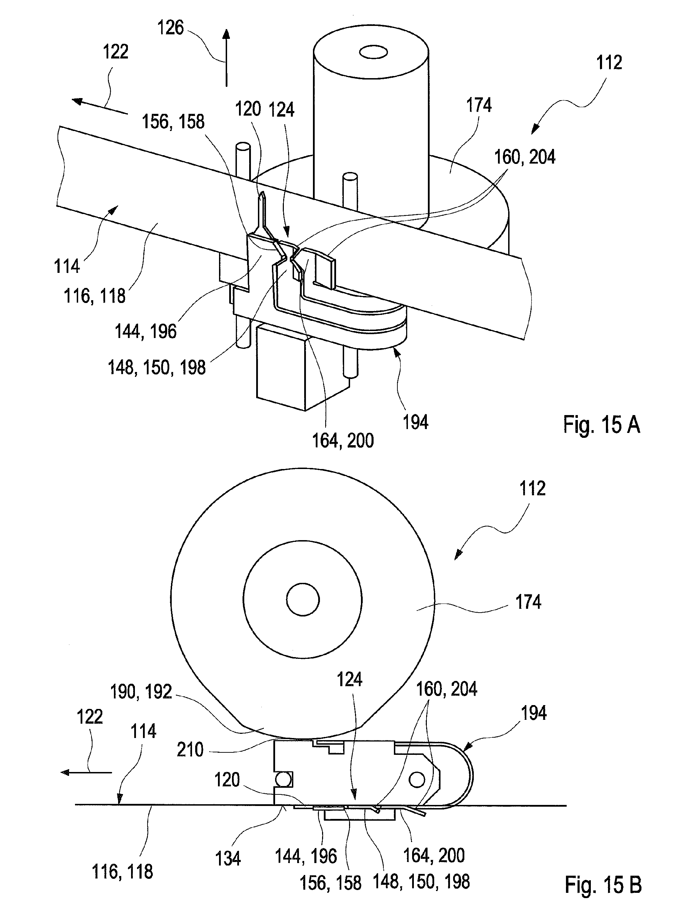

In den Figuren 15A und 15B ist eine den Figuren 11A und 11B entsprechende Situation nach einem Stechvorgang dargestellt. Die als Stoppelement 144 wirkende erste Zunge 196 wird durch die Rampe 190 bzw. Stufe 192 der Antriebsscheibe 174 über das in den Figuren 15A und 15B nicht mehr erkennbare Übertragungselement 210 angehoben, sodass die Lanzette 120 aus der Greifvorrichtung 112 befreit werden kann. Die Lanzette 120 kann während dieses Vorgangs zumindest teilweise durch den Niederhalter 150 gehalten werden, sodass diese von der ersten Zunge getrennt aus der Greifvorrichtung 112 gezogen werden kann.

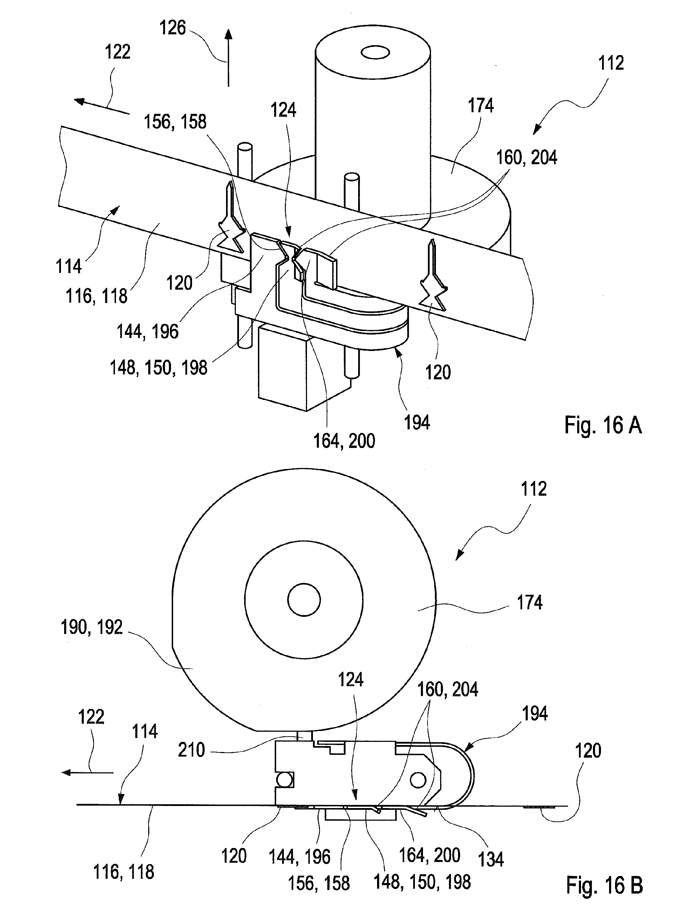

In den Figuren 16A und 16B ist schließlich eine Situation analog zur Situation in den Figuren 11A und 11B dargestellt. Eine gebrauchte Lanzette 120 ist nun aus der Greifvorrichtung 112 befreit, und eine neue Lanzette 120 kann in die Greifvorrichtung 112 transportiert werden. Die als Stopplasche und Stoppelement 144 wirkende erste Zunge 196 liegt in dieser Situation wieder an.

### Bezugszeichenliste

| | | | |
|---|---|---|---|
| 110 | Lanzettenvorrichtung | 168 | Zwischenraum |
| 112 | Greifvorrichtung | 170 | Haltebereich |
| 114 | Lanzettenband | 172 | Stechantrieb |
| 116 | Trägerelement | 174 | Antriebsscheibe |
| 118 | Trägerband | 176 | Greiferantriebsscheibe |
| 120 | Lanzette | 178 | Spannscheibe |
| 122 | Spulrichtung | 180 | Stechfeder |
| 124 | Applikationsposition | 182 | Zahnrad |
| 126 | Stechrichtung | 184 | Auslösemechanismus |
| 128 | Umlenkrollen | 186 | Fortsatz |
| 130 | Längserstreckungsrichtung | 188 | Rampe |
| 132 | Bandumlenkung | 190 | Rampe |
| 134 | Auflagefläche | 192 | Stufe |
| 136 | Greiferunterteil | 194 | Greiferblech |
| 138 | Führungsnut | 196 | erste Zunge |
| 140 | Trägerplatte | 198 | zweite Zunge |
| 142 | Greiferführung | 200 | dritte Zunge |
| 144 | Stoppelement | 202 | Fixierung |
| 146 | Aufnahme | 204 | Lasche |
| 148 | Positionierungselement | 206 | Lanzettenspitze |
| 150 | Niederhalter | 208 | Einschnürung |
| 152 | Greiferfeder | 210 | Übertragungselement |
| 154 | Greiferfeder | | |
| 156 | Anschlag | | |
| 158 | Anschlagskante | | |
| 160 | Rampe | | |
| 162 | Kraft | | |
| 164 | Gegengreifer | | |
| 166 | Kante | | |

## Patentansprüche

1. Greifvorrichtung (112) zum Einsatz in einer Lanzettenvorrichtung (110) zur Generierung einer Probe einer Körperflüssigkeit, wobei in der Lanzettenvorrichtung (110) eine Mehrzahl von Lanzetten (120) nacheinander auf einem Trägerelement (116) in einer Applikationsposition (124) bereitstellbar sind, wobei die Greifvorrichtung (112) eingerichtet ist, um in der Applikationsposition (124) jeweils eine Lanzette (120) zu erfassen und eine Stechbewegung mit der Lanzette (120) durchzuführen, wobei die Greifvorrichtung (112) mindestens ein Stoppelement (144) aufweist, wobei das Stoppelement (144) eingerichtet ist, um die Lanzette (120) in der Applikationsposition (124) zu stoppen und eine Weiterbewegung des Trägerelements (116) vorübergehend zu verhindern, wobei die Greifvorrichtung (112) weiterhin mindestens ein Positionierungselement (148) aufweist, wobei das Positionierungselement (148) eingerichtet ist, um die Lanzette (120) während der Stechbewegung in mindestens einer Richtung quer zu einer Stechrichtung (126) zu positionieren.

2. Greifvorrichtung (112) nach dem vorhergehenden Anspruch, wobei das Positionierungselement (148) mindestens einen Niederhalter (150) aufweist, wobei der Niederhalter (150) eingerichtet ist, um eine Kraft auf die Lanzette (120) senkrecht zu einer durch eine Längserstreckungsrichtung (130) des Trägerelements (116) und durch die Stechrichtung (126) gebildeten Ebene auszuüben.

3. Greifvorrichtung (112) nach einem der vorhergehenden Ansprüche, wobei die Greifvorrichtung (112) weiterhin ein Greiferunterteil (136) aufweist, wobei die Greifvorrichtung (112) eingerichtet ist, um das Trägerelement (116) zwischen dem Greiferunterteil (136) und dem Positionierungselement (148) zu führen, wobei das Positionierungselement (148) eingerichtet ist, um das Trägerelement (116) mit einer Kraft in Richtung des Greiferunterteils (136) zu beaufschlagen.

4. Greifvorrichtung (112) nach einem der vorhergehenden Ansprüche, wobei das Stoppelement (144) lösbar ausgestaltet ist und nach Durchführung des Stechvorgangs derart gelöst werden kann, dass eine Weiterbewegung des Trägerelements (116) wieder ermöglicht wird.

5. Greifvorrichtung (112) nach einem der vorhergehenden Ansprüche, wobei das Stoppelement (144) mindestens einen Anschlag (156) aufweist, wobei der Anschlag (156) benachbart zu dem Trägerelement (116) positionierbar ist und derart ausgestaltet ist, dass bei einer Bewegung des Trägerelements (116) eine Lanzette (120) an diesem Anschlag (156) gestoppt wird, wobei der Anschlag (156) in einer Richtung quer zu einer Längserstreckungsrichtung (130) des Trägerelements (116) beweglich ist, um das Stoppelement (144) zu lösen.

6. Greifvorrichtung (112) nach einem der vorhergehenden Ansprüche, weiterhin aufweisend mindestens einen Gegengreifer (164), wobei der Gegengreifer (164) eingerichtet ist, um derart mit dem Stoppelement (144) zusammenzuwirken, dass die Lanzette (120) formschlüssig gehaltert werden kann.

7. Greifvorrichtung (112) nach dem vorhergehenden Anspruch, wobei die Greifvorrichtung (112), insbesondere der Gegengreifer (164) und/oder die Positionierungselement (148), mindestens eine Rampe (160) aufweist, wobei die Rampe (160) eingerichtet ist, um ein Einführen der Lanzette (120) zwischen den Gegengreifer (164) und das Stoppelement (144) zu ermöglichen.

8. Greifvorrichtung (112) nach einem der vorhergehenden Ansprüche, wobei die Greifvorrichtung (112) weiterhin mindestens eine ortsfeste Greiferführung (142) aufweist, wobei zumindest das Stoppelement (144) und das Positionierungselement (148) und vorzugsweise der Gegengreifer (164) beweglich in der Greiferführung (142) gelagert sind.

9. Greifvorrichtung (112) nach einem der vorhergehenden Ansprüche, weiterhin umfassend mindestens eine Greiferfeder (152, 154), wobei die Greiferfeder (152, 154) eingerichtet ist, um das Stoppelement (144) und das Positionierungselement (148) und vorzugsweise den Gegengreifer (164) mit einer Kraft in Richtung des Trägerelements (116) zu beaufschlagen.

10. Greifvorrichtung (112) nach einem der vorhergehenden Ansprüche, wobei das Stoppelement (144) und das Positionierungselement (148) und vorzugsweise der Gegengreifer (164) zumindest teilweise als Federbauteile ausgestaltet sind.

11. Greifvorrichtung (112) nach dem vorhergehenden Anspruch, wobei das Stoppelement (144) und das Positionierungselement (148) und vorzugsweise der Gegengreifer (164) zumindest teilweise als Zungen (196, 198, 200) eines Federblechs ausgestaltet sind, wobei das Federblech eine als das Stoppelement (144) wirkende erste Zunge (196), eine als das Positionierungselement (148) wirkende zweite Zunge (198) und vorzugsweise eine der ersten Zunge (196) gegenüberliegende und als Gegengreifer (164) wirkende dritte Zunge (198) aufweist.

12. Lanzettenvorrichtung (110) zur Generierung einer Probe einer Körperflüssigkeit, wobei in der Lanzettenvorrichtung (110) eine Mehrzahl von Lanzetten (120) nacheinander auf einem Trägerelement (116) in einer Applikationsposition (124) bereitstellbar ist, wobei die Lanzettenvorrichtung (110) mindestens eine Greifvorrichtung (112) nach einem der vorhergehenden Ansprüche aufweist.

13. Lanzettenvorrichtung (110) nach dem vorhergehenden Anspruch, weiterhin umfassend mindestens einen Stechantrieb, wobei der Stechantrieb eingerichtet ist, um die Greifvorrichtung (112) zu der Stechbewegung anzutreiben, wobei der Stechantrieb eingerichtet ist, um das Stoppelement (144) nach der Stechbewegung zu lösen und die zuvor für die Stechbewegung verwendete Lanzette (120) wieder freizugeben.

14. Lanzettenvorrichtung (110) nach dem vorhergehenden Anspruch, wobei der Stechantrieb mindestens eine Erhöhung aufweist, insbesondere eine Rampe (190) und/oder eine Stufe (192), wobei die Erhöhung eingerichtet ist, um mindestens einen Anschlag (156) des Stoppelements (144) der Greifvorrichtung (112) quer zu einer Längserstreckungsrichtung (130) des Trägerelements (116) zu bewegen, um das Stoppelement (144) zu lösen.

15. Lanzettenvorrichtung (110) nach einem der beiden vorhergehenden Ansprüche, wobei der Stechantrieb mindestens eine Antriebsscheibe (174) aufweist.

16. Lanzettenvorrichtung (110) nach einem der vorhergehenden, eine Lanzettenvorrichtung (110) betreffenden Ansprüche, wobei die Lanzette (120) einen von der Greifvorrichtung (112) erfassbaren Haltebereich (170) aufweist, wobei der Haltebereich (170) mindestens eine äußere Kontur aufweist, die eine Beaufschlagung der Lanzette (120) mit einer Kraft parallel zur Stechbewegung ermöglicht, wobei das Stoppelement (144) und vorzugsweise der Gegengreifer (164) eine der äußeren Kontur des Haltebereichs (170) zumindest näherungsweise angepasste Innenkontur aufweisen.
